# FASCICULE DE BREVET EUROPEEN

(11) **EP 0 861 228 B1**
(45) Date de publication et mention de la délivrance du brevet: **23.08.2000**
(21) Numéro de dépôt: 96938291.0
(22) Date de dépôt: 08.11.1996
(51) Int. Cl.: C07C 237/06, C07K 5/06, A61K 47/48, C12N 15/87, C12N 15/88, A61K 48/00

(54) **LIPOPOLYMAMINES COMME AGENTS DE TRANSFECTION ET LEURS APPLICATIONS PHARMACEUTIQUES**
LIPOPOLYAMINE ALS TRANSFECTIONSMITTEL UND IHRE PHARMAZEUTISCHEN ANWENDUNGEN
LIPOPOLYAMINES AS TRANSFECTION AGENTS AND PHARMACEUTICAL USES THEREOF

(30) Priorité: 14.11.1995 FR 9513490
(43) Date de publication de la demande: 02.09.1998
(73) Titulaire: Aventis Pharma S.A., 92160 Antony (FR)
(72) Inventeur: BYK, Gérardo, F-94000 Créteil (FR); SCHERMAN, Daniel, F-75012 Paris (FR); SCHWARTZ, Bertrand, F-94700 Maisons-Alfort (FR); DUBERTRET, Catherine, F-92310 Sèvres (FR)
(86) Numéro de dépôt international: FR9601774
(87) Numéro de publication internationale: WO9718185

(56) Documents cités:
- EP-A- 0 394 111
- WO-A-94/05624
- WO-A-95/18863
- WO-A-96/25508

## Description

La présente invention concerne de nouveaux composés apparentés à la famille des lipopolyamines, des compositions pharmaceutiques les contenant, leurs applications pour la transfection in vivo et/ou in vitro d'acides nucléiques et leur procédé de préparation.

De nombreuses maladies génétiques sont associées à un défaut d'expression et/ou une expression anormale, c'est à dire déficiente ou excessive, d'un ou plusieurs acides nucléiques. La thérapie génique a pour principal objectif de corriger ce type d'anomalies génétiques par le biais de l'expression cellulaire in vivo ou in vitro de gènes clonés.

Aujourd'hui, plusieurs méthodes sont proposées pour la délivrance intracellulaire de ce type d'information génétique. L'une d'entre elles, en particulier, repose sur l'emploi de vecteurs chimiques ou biochimiques. Les vecteurs synthétiques ont deux fonctions principales, compacter l'ADN à transfecter et promouvoir sa fixation cellulaire ainsi que son passage à travers la membrane plasmique et, le cas échéant, à travers les deux membranes nucléaires.

Un progrès important a été accompli dans ce mode de transfection avec le développement d'une technologie basée sur l'emploi d'un lipide cationique. Il a ainsi été mis en évidence qu'un lipide cationique chargé positivement, le chlorure de N-[1-(2,3-dioleyloxy)propyl]-N,N,N-triméthylammonium (DOTMA), interférait, sous la forme de liposomes ou de petites vésicules, spontanément avec de l'ADN, qui est chargé négativement, pour former des complexes lipides-ADN, capables de fusionner avec les membranes cellulaires, et permettait ainsi la délivrance intracellulaire de l'ADN. Toutefois, bien que cette molécule soit efficace au niveau de la transfection, elle présente le désavantage d'être non biodégradable et de posséder un caractère toxique à l'égard des cellules.

Depuis le DOTMA, d'autres lipides cationiques ont été développés sur ce modèle de structure : groupe lipophile associé à un groupement amino via un bras dit "spacer". Parmi ceux-ci, on peut plus particulièrement citer ceux comprenant à titre de groupement lipophile deux acides gras ou un dérivé du cholestérol, et comportant, en outre, le cas échéant, à titre de groupement amino, un groupement d'ammonium quaternaire. Les DOTAP, DOBT ou le ChOTB peuvent notamment être cités à titre représentatifs de cette catégorie de lipides cationiques. D'autres composés, comme les DOSC et ChOSC, se caractérisent par la présence d'un groupement choline à la place du groupement d'ammonium quaternaire. En général, l'activité transfectante de ces composés demeure toutefois assez faible.

Une autre catégorie de lipides cationiques, les lipopolyamines, a également été décrite en particulier dans le brevet EP 349 111. Au sens de la présente invention, le terme lipopolyamine désigne une molécule amphiphile comprenant au moins une région hydrophile polyamine associée par une région dite spacer à une région lipophile. La région polyamine des lipopolyamines, chargée cationiquement, est capable de s'associer de manière réversible avec l'acide nucléique, chargé négativement. Cette interaction compacte fortement l'acide nucléique. La région lipophile rend cette interaction ionique insensible au milieu externe, en recouvrant la particule nucléolipidique formée d'une pellicule lipidique.Dans ce type de composés, le groupement cationique peut être représenté par le radical L-5carboxyspermine qui contient quatre groupements d'ammonium, deux primaires et deux secondaires. Les DOGS et DPPES décrits dans EP 394 111 en font notamment partie. Ces lipopolyamines sont tout particulièrement efficaces pour la transfection de cellules endocrines primaires.

En fait, un agent de transfection synthétique idéal devrait présenter un haut niveau de transfection, et ce pour un large spectre de cellules, posséder une toxicité nulle ou à défaut une toxicité très minimisée aux doses d'utilisation, et enfin, être biodégradable pour s'affranchir de tout effet secondaire au niveau des cellules traitées.

La présente invention a précisément pour objet de proposer de nouvelles lipopolyamines, originales de par leur fraction polyamine, et qui soient susceptibles d'être utilisés efficacement dans la transfection in vitro et ou in vivo de cellules et notamment pour la vectorisation d'acides nucléiques.

La présente invention a pour premier objet des lipopolyamines, sous forme D, L ou LD et leurs sels caractérisées en ce qu'elles sont représentées par la formule générale I 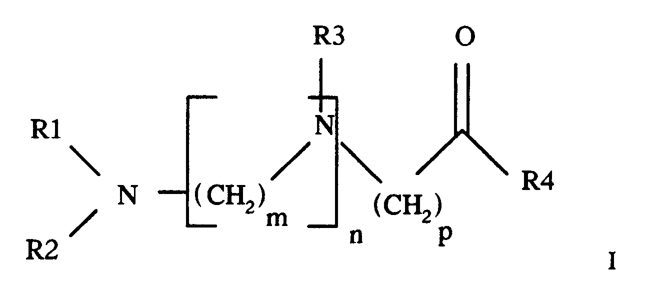 Dans laquelle :
R1, R2 et R3 représentent indépendamment l'un de l'autre un atome d'hydrogène ou un groupement -(CH2)q-NRR' avec
q pouvant varier entre 1, 2, 3, 4, 5 et 6 ceci de manière indépendante entre les différents groupements R1, R2 et R3 et
R et R' représentant indépendamment l'un de l'autre un atome d'hydrogène ou un groupement -(CH₂)q'-NH₂, q' pouvant varier entre 1, 2, 3, 4, 5 et 6 ceci de manière indépendante entre les différents groupements R et R',
m, n et p représentent, indépendamment l'un de l'autre, un nombre entier pouvant varier entre 0 et 6 avec lorsque n est supérieur à 1, m pouvant prendre des valeurs différentes et R3 des significations différentes au sein de la formule générale I et
R4 représente un groupement de formule générale II 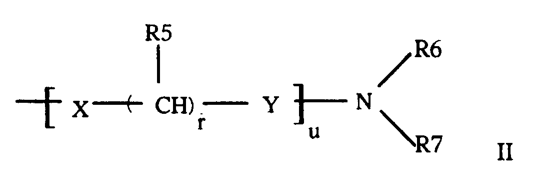 dans laquelle:
R6 et R7 représentent indépendamment l'un de l'autre un atome d'hydrogène ou un radical aliphatique, saturé ou non, en C10 à C22 avec au moins l'un des deux groupements étant différent de l'hydrogène,
u est un nombre entier choisi entre 0 et 10 avec lorsque u est un entier supérieur à 1 R5, X, Y et r pouvant avoir des significations différentes au sein des différents motifs [ X-(CHR5)r-Y]
X représente un atome d'oxygène, de soufre ou un groupement amine monoalkylé ou non,
Y représente un groupement carbonyle ou un groupement méthylène
R5 représente un atome d'hydrogène ou une chaine latérale d'acide aminé naturel, le cas échéant substituée et
r représente un entier variant entre 1 et 10 avec lorsque r est égal à 1, R5 représentant une chaine latérale d'acide aminé naturel et lorsque r est supérieur à 1, R5 représentant un atome d'hydrogène.

A titre de chaine latérale d'acide aminé naturel on entend notamment désigné au sens de l'invention des chaines contenant des motifs amidinium comme par exemple la chaine latérale de l'arginine. Comme il l'est énoncé précédemment cette chaine peut être substituée par des groupements aliphatiques saturés ou insaturés, linéaires, ramifiés ou cycliques en C1 à C24 tels par exemple des radicaux cholestéryle, arachidonyle ou rétinoyle, des groupements mono- ou poly-aromatiques tels par exemple des dérivés benzyloxycarbonyles, benzyle esters, rhodaminyle substitués ou non.

Ces nouveaux produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique ou oxalique) ou avec les bases minérales (soude, potasse, lithine, chaux) ou organiques (amines tertiaires comme la triéthylamine, la pipéridine, la benzylamine).

A titre représentatif des composés selon l'invention on peut plus particulièrement citer les composés de sous-formules générales suivantes: 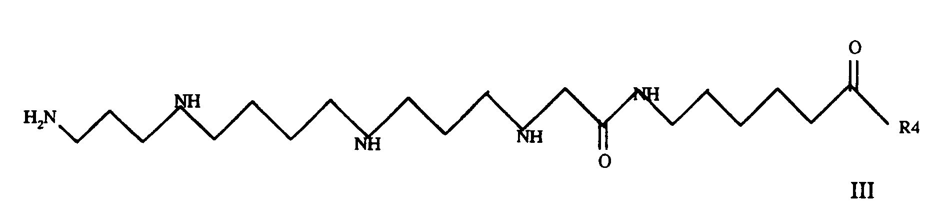 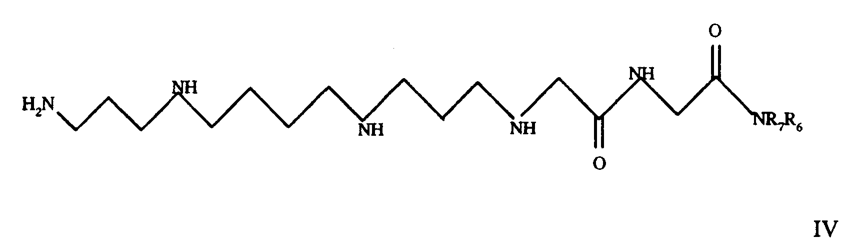 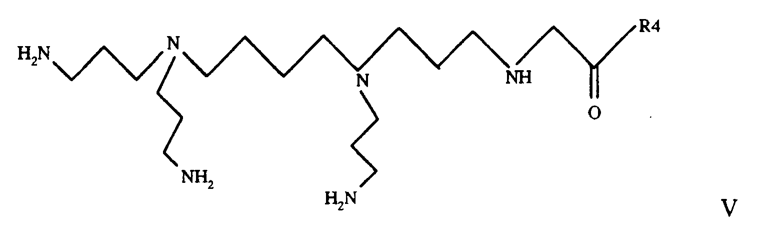 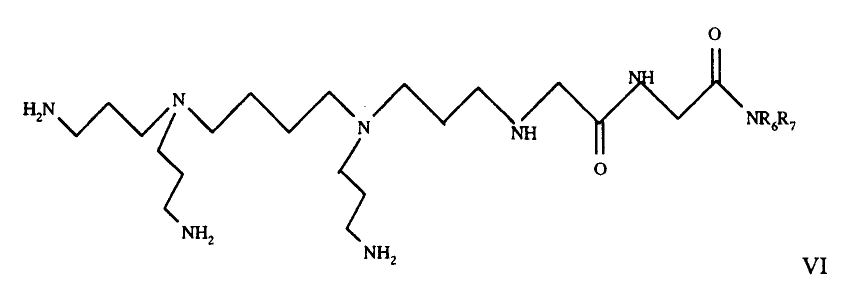 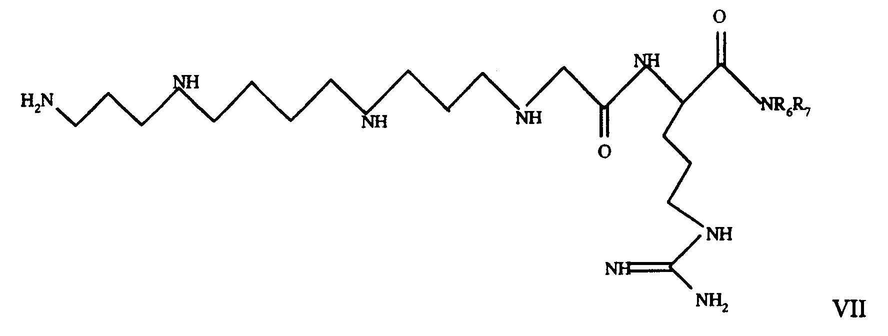 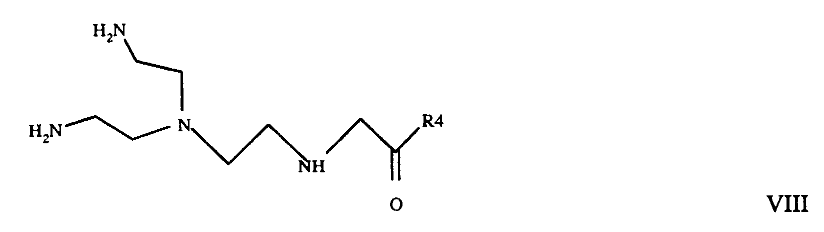 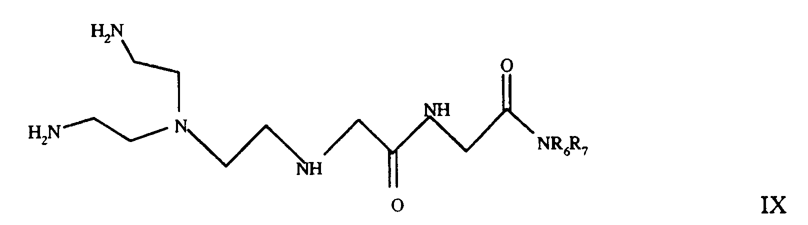 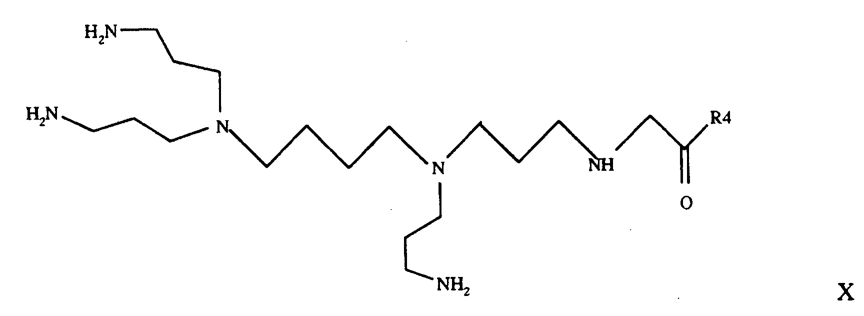 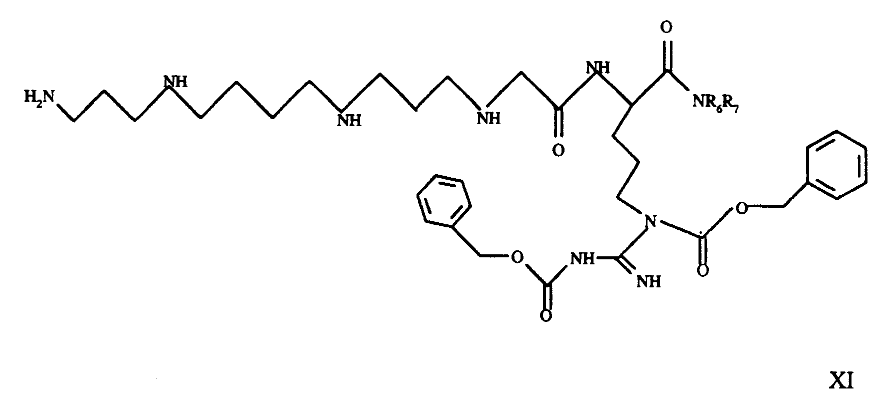 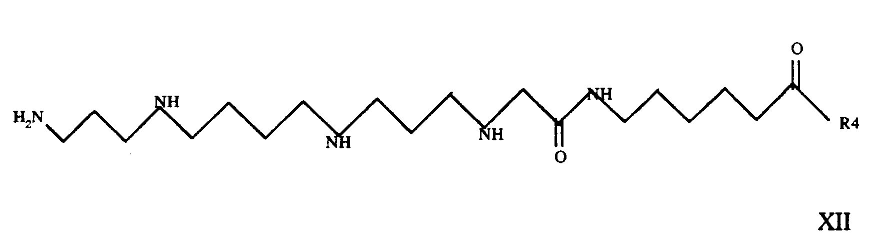 dans lesquelles R4, R6 et R7 possèdent les définitions précédentes. Préférentiellement, R4 y représente un groupement NR6R7 avec R6 et R7 figurant dans les sous formules III à XII un groupement identique choisi parmi (CH₂)₁₇ CH₃, (CH₂)₁₁ CH₃, (CH₂)₁₃ CH₃ ou (CH₂)₁₂ CH₃.

Dans un mode de réalisation particulièrement avantageux, les composés revendiqués comprennent en outre un élément de ciblage permettant d'orienter le transfert de l'acide nucléique auquel ils sont associés. Cet élément de ciblage est de préférence incorporer, sur le composé de formule générale I, au niveau de la chaîne latérale d'acide aminé figurée par le substituant R5. Plus préférentiellement, l'élément de ciblage est lié, de manière covalente ou non covalente, au composé selon l'invention.

Il peut s'agir d'un élément de ciblage extracellulaire, permettant d'orienter le transfert de l'acide nucléique vers certains types cellulaires ou certains tissus souhaités (cellules tumorales, cellules hépatiques, cellules hématopoiétiques, etc). A ce titre, il peut s'agir d'un ligand de récepteur cellulaire présent à la surface du type cellulaire ciblé comme par exemple un sucre, un folate, une transférrine, une insuline, une protéine asialo-orosomucoïde ou toute molécule bioactive reconnue par des récepteurs extracellulaires. Il peut également s'agir d'un élément de ciblage intracellulaire, permettant d'orienter le transfert de l'acide nucléique vers certains compartiments cellulaires privilégiés (mitochondries, noyau, etc) comme par exemple, une séquence signal de localisation nucléaire, (nls), qui privilégie l'accumulation de l'ADN transfecté au sein du noyau.

Plus généralement, les éléments de ciblage susceptibles d'être mis en oeuvre dans le cadre de l'invention incluent les sucres, les peptides, les oligonucléotides, les stéroïdes ou les lipides. Préférentiellement, il s'agit de sucres et/ou peptides tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion telles que les intégrines. On peut également citer le récepteur de la transferrine, des lipides HDL et des LDL. L'élément de ciblage peut également être un sucre permettant de cibler des lectines tels que les récepteurs asialoglycoprotéiques, ou encore un fragment Fab d'anticorps permettant de cibler le récepteur du fragment Fc des immunoglobulines.

De la même façon, il est possible d'envisager l'association à un composé de formule générale I d'un agent marqueur de type biotine, rhodamine, folate par exemple sur la chaîne latérale d'acide aminé R5. Cet agent marqueur peut également être une séquence peptidique ou pseudopeptidique linéaire ou cyclique comportant l'épitope Arg-Gly-Asp de reconnaissance des récepteurs primaires et/ou secondaires des protéines d'adhésion du type intégrines.

A titre illustratif des lipopolyamines revendiquées, on peut plus particulièrement citer les composés suivants, décrits de manière plus détaillée dans les exemples ci-après:
H₂N(CH₂)₃NH(CH₂)₄NH(CH₂)₃NHCH₂COGlyN[(CH₂)₁₇-CH₃]₂
H₂N(CH₂)₃NH(CH₂)₄NH(CH₂)₃NHCH₂CON[(CH₂)₁₇-CH₃]₂
H₂N(CH₂)₃NH(CH₂)₄NH(CH₂)₃NHCH₂COArgN[(CH₂)₁₇-CH₃]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(rhodamine)N[(CH₂)₁₇-CH₃]₂
H₂N(CH₂)₃NH(CH₂)₄NH(CH₂)₃NHCH₂COLys(biotinyl)N[(CH₂)₁₇-CH₃]₂
{H₂N(CH₂)₃}₂N(CH₂)₄N{(CH₂)₃NH₂}(CH₂)₃NHCH₂COGlyN[(CH₂)₁₇-CH₃]₂
{H₂N(CH₂)₃}₂N(CH₂)₄N{(CH₂)₃NH₂}(CH₂)₃NHCH₂CON[(CH₂)₁₇-CH₃]₂
{H₂N(CH₂)₂}₂N(CH₂)₂NHCH₂COGlyN[(CH₂)₁₇-CH₃]₂
{H₂N(CH₂)₂}₂N(CH₂)₂NHCH₂CON[(CH₂)₁₇-CH₃]₂
NH₂(CH₂)₃NH(CH₂)₄N[(CH₂)₃NH₂]CH₂COGlyN[(CH₂)₁₇CH₃]₂
NH₂(CH₂)₃NH(CH₂)₄NH(CH₂)₃NHCH₂COLysN[(CH₂)₁₇CH₃]₂
NH₂(CH₂)₃NH(CH₂)₄NH(CH₂)₃NHCH₂COLys[Cl-Z]N[(CH₂)₁₇CH₃]₂
NH₂(CH₂)₃NH(CH₂)₄NH(CH₂)₃NHCH₂COLys[CHO]N[(CH₂)₁₇CH₃]₂
NH₂(CH₂)₃NH(CH₂)₄NH(CH₂)₃NHCH₂COLys[Cholesteryl]N[(CH₂)₁₇CH₃]₂
NH₂(CH₂)₃NH(CH₂)₄NH(CH₂)₃NHCH₂COLys[Arachidonyl]N[(CH₂)₁₇CH₃]₂
NH₂(CH₂)₃NH(CH₂)₄NH(CH₂)₃NHCH₂COGluN[(CH₂)₁₇CH₃]₂
NH₂(CH₂)₃NH(CH₂)₄NH(CH₂)₃NHCH₂COGlu[N(CH₃)₂]N[(CH₂)₁₇CH₃]₂
NH₂(CH₂)₃NH(CH₂)₄NH(CH₂)₃NHCH₂COGlu[O-Bz]N[(CH₂)₁₇CH₃]₂
NH₂(CH₂)₃NH(CH₂)₄NH(CH₂)₃NHCH₂COGlu[Galactosamide]N[(CH₂)₁₇CH₃]₂
NH₂(CH₂)₃NH(CH₂)₄NH(CH₂)₃NHCH₂COGlu[Glucosamide]N[(CH₂)₁₇CH₃]₂
NH₂(CH₂)₃NH(CH₂)₄NH(CH₂)₃NHCH₂COGlu[Mannosamide]N[(CH₂)₁₇CH₃]₂
NH₂(CH₂)₃NH(CH₂)₄NH(CH₂)₃NH(CH₂)₃CON[(CH₂)₁₇CH₃]₂
NH₂(CH₂)₃NH(CH₂)₄NH(CH₂)₃NHCH₂CONH(CH₂)₅CON[(CH₂)₁₇CH₃]₂
NH₂(CH₂)₃NH(CH₂)₄NH(CH₂)₃NHCH₂COGlyN[(CH₂)₁₁CH₃]₂
NH₂(CH₂)₃NH(CH₂)₄NH(CH₂)₃NHCH₂COGlyN[(CH₂)₁₂CH₃]₂ et
NH₂(CH₂)₃NH(CH₂)₄NH(CH₂)₃NHCH₂COGlyN[(CH₂)₁₃CH₃]₂.

A titre de composés particulièrement représentatifs de la présente invention on peut plus notamment cité dont la formule générale est représentée ci-après.

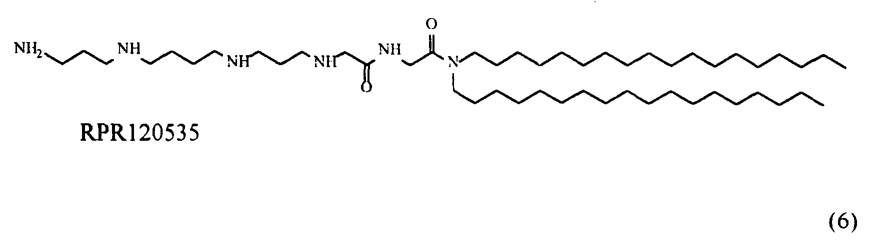

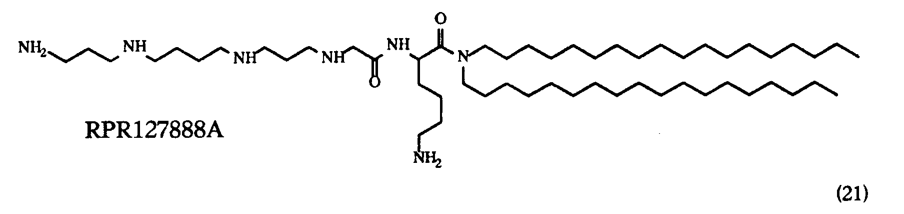

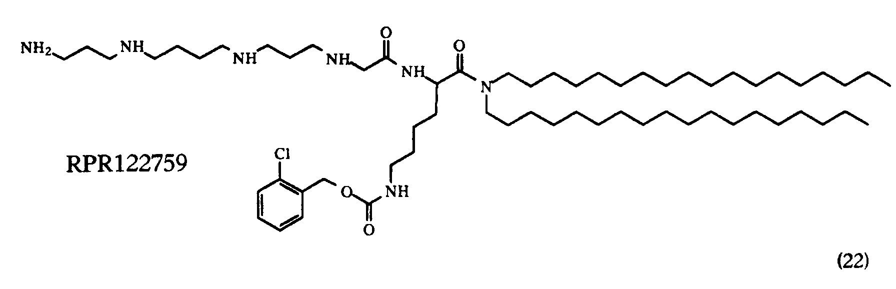

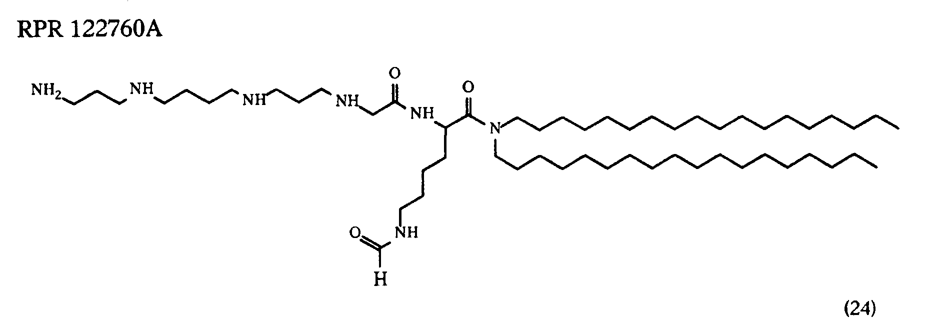

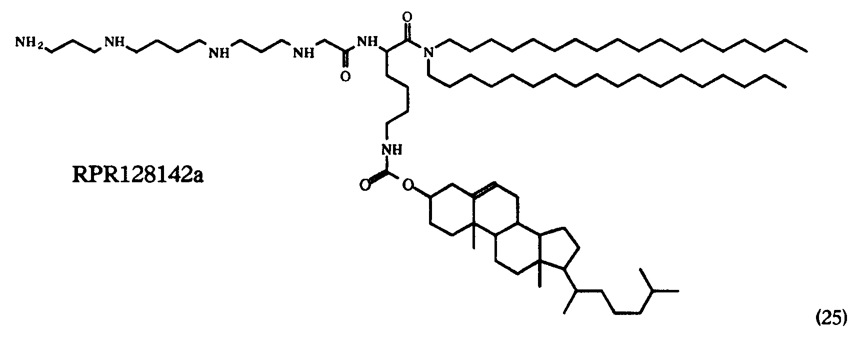

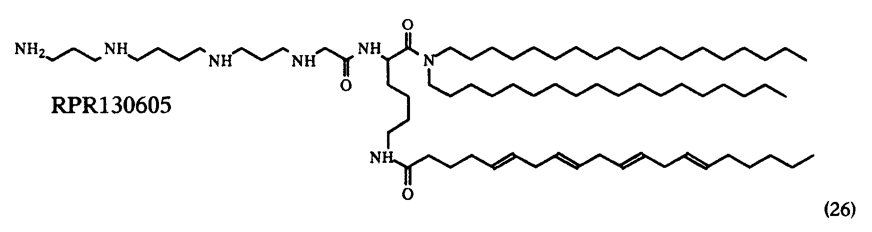

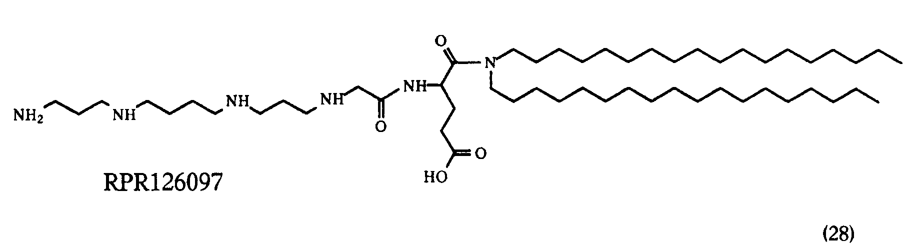

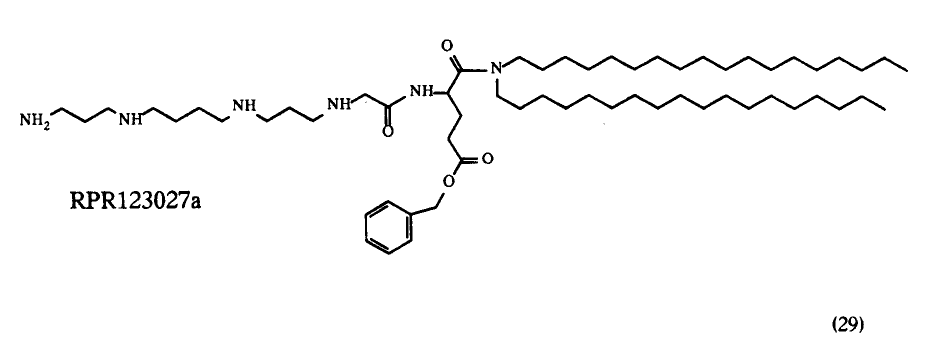

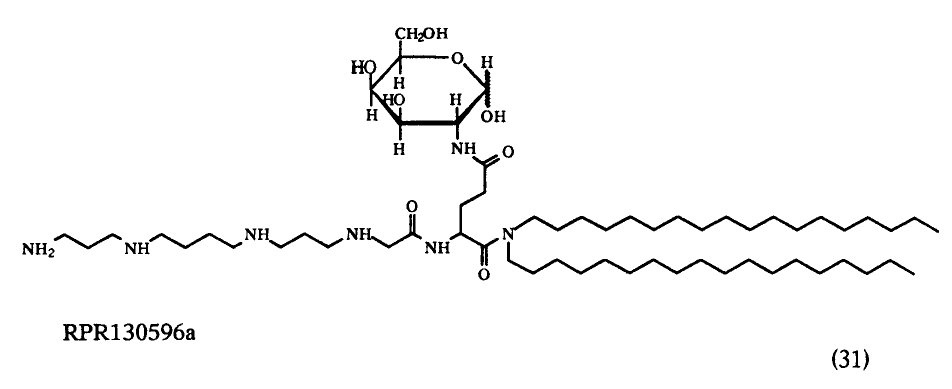

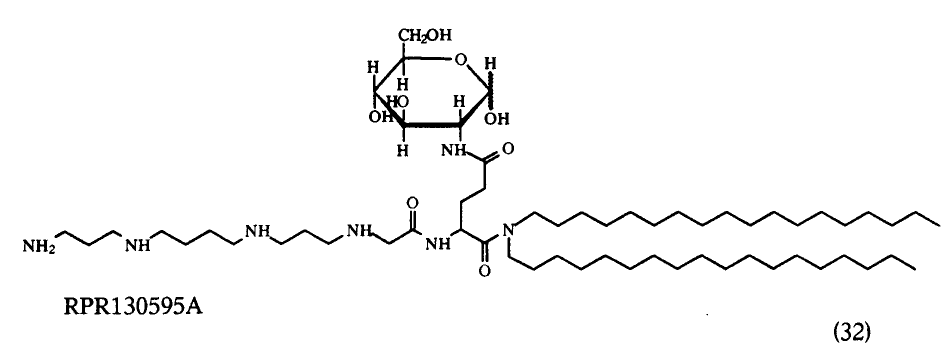

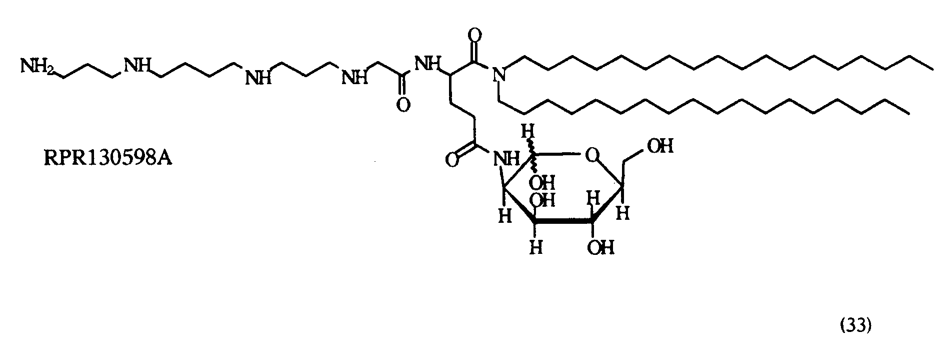

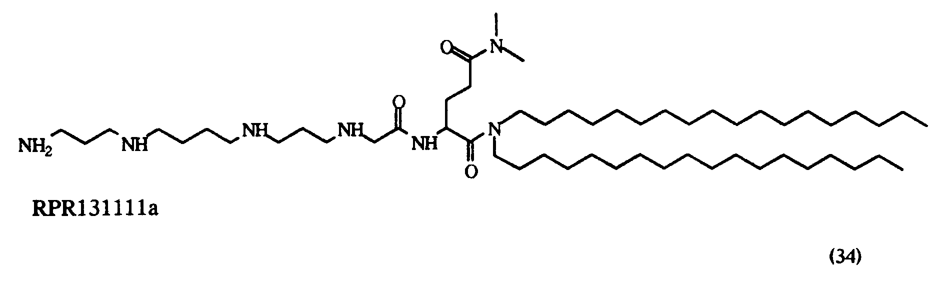

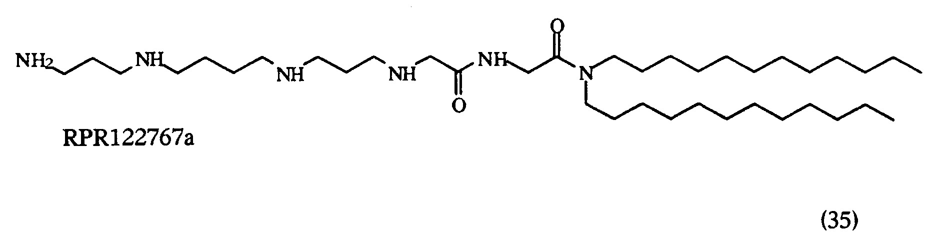

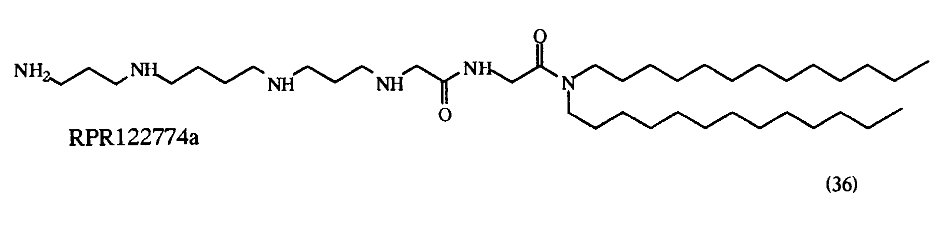

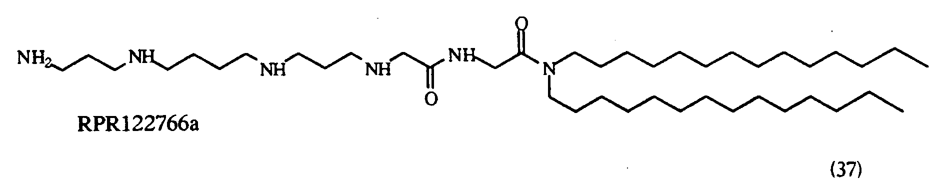

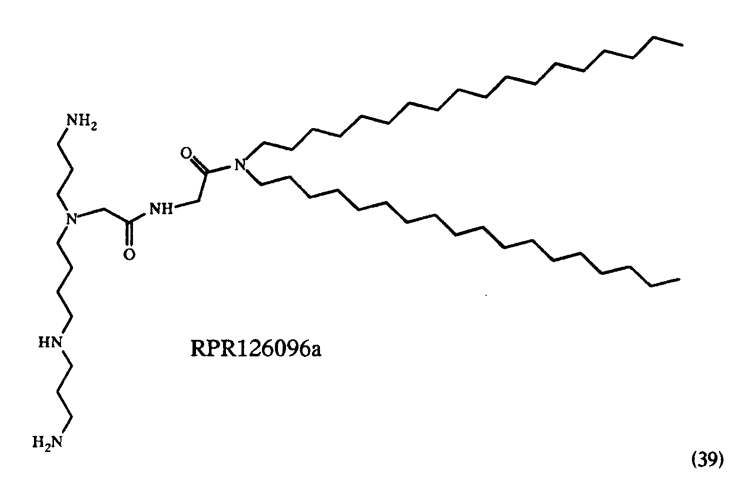

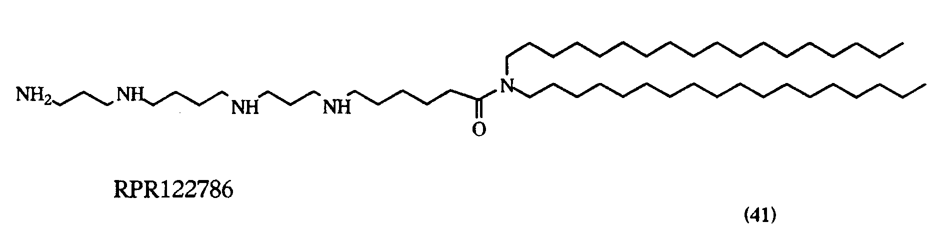

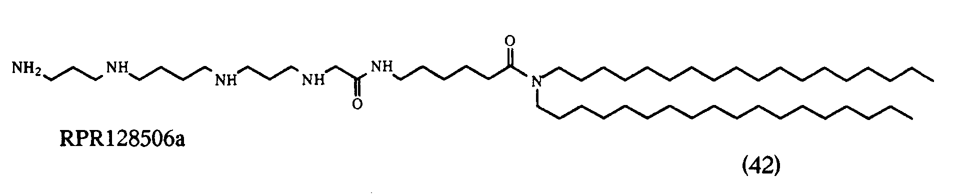

Dans le cadre de la présente invention, il a en outre été développé une méthodologie originale en phase solide pour préparer des polyamines fonctionnalisées assymétriques dont dérivent les lipopolyamines selon l'invention.

Classiquement, l'accès aux polyamines fonctionnalisées assymétriques est limité par la nécessité d'introduire sélectivement des modifications dans des polyamines symétriques linéaires ou branchées. Les différences chimiques entre les groupes aminés primaires et secondaires d'une polyamine requièrent une grande sélectivité pour effectuer des réactions telles que des alkylations, des additions de type Michael ou des acylations. De plus, la sélectivité imposée par de telles différences chimiques n'est pas compatible avec une alkylation sélective dans un groupe de plusieurs amines primaires et secondaires, comme on en rencontre dans les polyamines. La réponse classique à de telles restrictions est de construire les polyamines fonctionnalisées assymétriques à partir de blocs monomères portant d'un côté un groupe aminé susceptible d'être "polyaminé" et de l'autre côté la fonction asymétrique convenablement protégée. Cette approche nécessite donc une stratégie de synthèse fastidieuse en plusieurs étapes et en particulier une protection orthogonale des groupes fonctionnels.

La méthode développée dans le cadre de la présente invention, a précisément pour objectif de s'affranchir des inconvénients rencontrés jusqu'alors avec ce type de synthèse. Elle a pour net avantage de conduire commodément et rapidement à des polyamines qui sont fonctionnalisées sélectivement sur un unique groupe aminé primaire par alkylation ou alkylation réductive des polyamines symétriques.

Le principe du procédé revendiqué est fondé sur l'emploi d'une méthode de synthèse en phase solide pour favoriser une réaction bimoléculaire entre le réactif alkylant et la polyamine, ce qui évite la polyalkylation de celle-ci. Plus précisément, la présente invention se rapporte à un procédé caractérisé en ce qu'il met en oeuvre le couplage d'au moins une fraction lipidique à au moins une fraction polyamine assymétrique, ladite fraction polyamine ayant au préalable été obtenue par réaction bimoléculaire entre un agent alkylant lié covalemment à un support solide et une polyamine symétrique. Selon cette approche, le réactif alkylant est lié covalemment à un support polymère par estérification ou amidation. La polyamine symétrique réagit avec l'agent alkylant en phase solide par une réaction bimoléculaire qui mène à la polyamine asymétrique mono-fonctionnalisée attachée au support. Les amines libres du produit sont habituellement protégées en phase solide par des groupes protecteurs de type BOC ou Z, et finalement les produits sont clivés du support de phase solide. Ces acides polyaminés sont, comme il convient, couplés aux fractions lipidiques pour fournir les agents transfectants recherchés. Pour ce couplage, il est possiblede mettre en oeuvre des agents de couplage peptidique usuels comme BOP, Pybop, BopCl et DCC par exemple. La méthodologie permet également d'assembler l'agent transfectant entier sur le support solide avec l'introduction éventuelle de peptides traceurs, de sucres, ou de sondes fluorescentes dans les molécules. Bien entendu, il s'avère possible de procéder à ce type de greffage sur la lipopolyamine libre.

La faisabilité de la méthode a été démontrée par la synthèse de plusieurs acides polyaminés linéaires ou branchés assymétriques et fonctionnalisés.

La présente invention a également pour objet toute application thérapeutique des lipopolyamines telles que décrites précédemment, soit directement, soit au sein de compositions pharmaceutiques.

Comme explicité précédemment, les composés de formule générale I s'avèrent tout particulièrement intéressants pour la transfection in vitro et in vivo d'acides nucléiques. Ils compactent efficacement l'ADN et présentent avantageusement une toxicité très réduite.

Pour obtenir un effet maximal des compositions de l'invention, les proportions respectives du composé de formule générale I et de l'acide nucléique sont de préférence déterminées de manière à ce que le rapport R, charges positives de la lipopolyamine considérée par charges négatives dudit acide nucléique soit optimal. Ce rapport optimal variant en particulier selon le mode d'utilisation à savoir in vivo ou in vitro et selon le type cellulaire à transfecter, il est optimisé au cas par cas. Cette optimisation relève de la compétence de l'homme de l'art.

Dans les compositions pharmaceutiques de la présente invention, le polynucléotide peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques d'oligonucléotides modifiés ou non. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent par ailleurs être incorporés dans des vecteurs, tels que des vecteurs plasmidiques.

Concernant plus particulièrement les acides désoxyribonucléiques, ils peuvent être simple ou double brin de même que des oligonuléotides courts ou des séquences plus longues. Ces acides désoxyribonucléiques peuvent porter des gènes thérapeutiques, des séquences régulatrices de la transcription ou de la réplication, des séquences antisens modifiées ou non, des régions de liaison à d'autres composants cellulaires, etc.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc la dystrophine ou une minidystrophine (FR 9111947), la protéine CFTR associée à la mucoviscidose, les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholesterol et la protéine de transfert des phospholipides, une protéine de liaisons des HDL ou encore un récepteur choisi par exemple parmi les récepteurs LDL, récepteurs des chylomicrons-remnants et les récepteurs scavenger.etc.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus, d'autres virus ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes EIA, MLP, CMV, RSV, etc. En outre, ces séquences d'expression peuvent être modifiées par addition de séquences d'activation, de régulation, etc. Il peut aussi s'agir de promoteur, inductible ou répressible.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Dans un autre mode de mise en oeuvre, la présente invention concerne des compositions comprenant un acide nucléique, une lipopolyamine telle que revendiquée et un adjuvant capable de s'associer au complexe lipopolyamine/acide nucléique et d'en améliorer le pouvoir transfectant. La demanderesse a en effet montré que le pouvoir transfectant des lipopolyamines peut être de manière inattendue augmenté en présence de certains adjuvants (lipides, peptides ou protéines par exemple), capables de s'associer au complexe lipopolyamine/acide nucléique.

Dans cette optique, les compositions de l'invention peuvent comprendre comme adjuvant, un ou plusieurs lipides neutres. De telles compositions sont particulièrement avantageuses, notamment lorsque le rapport R est faible. La demanderesse a en effet montré que l'addition d'un lipide neutre permet d'améliorer la formation des particules nucléolipidiques et, de manière surprenante, de favoriser la pénétration de la particule dans la cellule en déstabilisant sa membrane.

Plus préférentiellement, les lipides neutres utilisés dans le cadre de la présente invention sont des lipides à 2 chaînes grasses.

De manière particulièrement avantageuse, on utilise des lipides naturels ou synthétiques, zwitterioniques ou dépourvus de charge ionique dans les conditions physiologique. Il peuvent être choisis plus particulièrement parmi la dioleoylphosphatidyléthanolamine (DOPE), l' oléoyl-palmitoylphosphatidyléthanolamine (POPE), les di-stéaroyl, -palmitoyl, -mirystoyl phosphatidyléthanolamines ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Très récemment, la demanderesse a démontré qu'il était également particulièrement avantageux d'employer à titre d'adjuvant, un composé intervenant ou non directement au niveau de la condensation dudit acide nucléique ( WO96/25508).

La présence d'un tel composé, au sein d'une composition transfectante à base d'une lipopolyamine, permettait de diminuer considérablement la quantité en cet agent, avec les conséquences bénéfiques qui en découlent sur le plan toxicologique, sans porter un préjudice quelconque à l'activité transfectante de ladite composition. Au contraire, celle-ci possède avantageusement un niveau de transfection supérieur.

Par composé intervenant au niveau de la condensation de l'acide nucléique, on entend définir un composé compactant, directement ou non, l'acide nucléique. Plus précisément, ce composé peut soit agir directement au niveau de l'acide nucléique à transfecter soit intervenir au niveau d'un composé annexe qui lui est directement impliqué dans la condensation de cet acide nucléique. De préférence, il agit directement au niveau de l'acide nucléique.

Selon un mode de réalisation préféré, cet agent intervenant au niveau de la condensation de l'acides nucléique est constitué, en tout ou partie, de motifs peptidiques (KTPKKAKKP) et/ou (ATPAKKAA), le nombre des motifs pouvant varier entre 2 et 10. Dans la structure du composé selon l'invention, ces motifs peuvent être répétés de manière continue ou non. C'est ainsi qu'ils peuvent être séparés par des liens de nature biochimique, par exemple un ou plusieurs acides aminés ou de nature chimique. Un tel agent peut également dériver en tout ou partie d'une histone, d'une nucléoline, d'une protamine et/ou de l'un de leurs dérivés.

Préférentiellement, les compositions de l'invention comprennent de 0,01 à 20 équivalents d'adjuvant pour un équivalent d'acides nucléiques en poids/poids et, plus préférentiellement, de 0,5 à 5.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus ou les voies circulatoires, soit d'un traitement de cellules en culture suivi de leur réimplantation in vivo, par injection ou greffe.

La présente invention fournit ainsi une méthode particulièrement avantageuse pour le traitement de maladies, comprenant l'administration in vivo ou in vitro d'un acide nucléique apte à corriger ladite maladie associé à un composé de formule générale I dans les conditions définies ci-avant. Plus particulièrement, cette méthode est applicable aux maladies résultant d'une déficience en un produit protéique ou nucléique et l'acide nucléique administré code pour ledit produit protéique ou contient ledit produit nucléique.

Elle s'étend à toute utilisation d'une lipopolyamine selon l'invention pour la transfection in vivo ou in vitro de cellules.

La présente invention sera plus complètement décrite à l'aide des exemples et figures qui suivent, qui doivent être considérés comme illustratifs et non limitatifs.

### FIGURES

Figure (1): Mesure de l'efficacité de transfection après traitement de cellules NIH 3T3 (cellules embryonnaires de souris - fibroblastes) par différents lipides cationiques.

Figure (2) : Mesure de l'efficacité de transfection après traitement de cellules SMC lapin (culture primaire de cellules musculaires lisses d'aorte de lapin) par différents lipides cationiques.

Figure (3) : Mesure de l'efficacité de transfection après traitement de cellules 3LL (carcinome pulmonaire de Lewis) par différents lipides cationiques.

Figure (4) : Mesure de l'efficacité de transfection après traitement de cellules NIH 3T3 (cellules embryonnaires de souris - fibroblastes) par différents lipides cationiques.

Figure (5) : Effet de la concentration en DOPE sur l'efficacité de transfection de cellules 3LL.

Figure (6) : Transfection de cellules NIH 3T3 avec des quantités variables d'ADN et un ratio nanomoles lipofectant/µg d'ADN constant.

### ABREVIATIONS ET SYMBOLES

- AcOEt :: Acétate d' éthyle
- BOC: t-Butoxycarbonyl
- BOP :: Benzotriazol-1-yloxytris (diméthylamino) phosphonium hexafluorophosphate
- DCC :: Dicyclohexylcarbodiimide
- DCU :: Dicyclohexylurée
- DMAP :: 4-Diméthylaminopyridine
- DMF :: Diméthylformamide
- DMSO :: Diméthyle sulfoxide
- DODA :: Dioctadécylamine
- EP :: Ether de pétrole
- EtOH :: Ethanol
- NEt₃ :: Triéthylamine
- Rf :: Coefficient de rétention frontal
- TFA :: Acide trifluoroacétique
- THF :: Tétrahvdrofurane
- TMS :: Tétraméthylsilane
- UV :: Ultra-Violets
- SPPS :: Solid Phase Peptide Synthesis
- CLHP :: Chromatographie Liquide à Haute Pression
- Z :: Benzyloxycarbonyl
- CIZ :: p-Chlorobenzyloxycarbonyl

### A- MATERIELS ET METHODES POUR LES SYNTHESES CHIMIQUES

### 1 MATERIEL

### a) Composés

- Les polyamines de départ sont disponibles commercialement, par exemple: spermidine, spermine, tris (2-aminoéthyle) amine, phénylène-diamine, diaminoéthane (propane, butane, pentane, hexane, etc.), ou peuvent être synthétisées par des méthodes classiques, par exemple par cyanoéthylation exhaustive d'amines disponibles dans le commerce telles que les diamino-éthane (propane, butane, pentane, hexane, etc.) amine, spermidine, spermine pour donner des polyamines branchées.
- Les agents alkylants sont choisis en fonction de la méthode d'alkylation comme suit: Pour une alkylation classique: l'acide bromoacétique, les acides ω-halogéno carboxyliques.

Pour une alkylation réductive: un acide ω-aldéhyde-carboxylique, tel que l'acide glyoxalique, le semi-aldéhyde succinique,etc., ou un céto-acide tel que l'acide acéto-acétique ou l'acide pyruvique, etc.
- Les polymères utilisés sont des résines disponibles dans le commerce pour la synthèse peptidique en phase solide (synthèse de Merrifield), par exemple la résine d'O-Chlorotrityl chlorure, la résine HMP, qui fournissent des produits portant des fonctions acides libres ou une résine de type Rink. Les acides polyaminés peuvent être synthétisés directement sur un peptide présynthétisé sur la phase solide et portant une fonction bromo-alkyl ou un acide w-aldéhyde.
- Les dioctadecylamine, triethylamine, trifluoroacetique, BOP, DMAP, chloroformate de benzyle de chez Aldrich.sont des produits commerciaux. Les solutions de NaCl et NaHCO₃ sont saturées; la solution de KHSO₄ est de 0.5 M

### b) Mesures Physiques.

Les spectres de RMN Proton ont été enregistrés sur des spectrométres Bruker 400 et 600 MHZ.

Les spectres de masse ont été réalisés sur un API-MS/III.

### c) Techniques de chromatographie

Les analyses CLHP sont réalisées sur un appareil Merck-Hitachi équipé d'un autosampler AS-2000A, de une pompe inteligent L-6200A et de un détecteur UV-vis L-4000 avec longueur d'onde réglable mise à 220 nm pour séparations analitiques et à 235nm pour séparations préparatives. Les colonnes pour les séparations analytiques sont des colonnes BU-300 aquapore Butyl 7m, 300 A 300x4.6 mm. de chez Perkin-Elmer et pour les séparations préparatives des colonnes Biosil C18 HL 90-10 250x10 mm de chez Biorad. Les phases mobiles sont H₂O (0.1 % TFA) et Acétonitrile (0.1% TFA). Le débit pour les analyses analytiques est réglé à 1 ml/min et pour les préparatives à 4 ml/min.

Les chromatographies sur couche mince (CCM) ont été effectuées sur des plaques de gel de silice Merck de 0,2 mm d' épaisseur.

.Les chromatographies sur colonne ont été effectuées sur gel de silice 60 Merck de granulométrie 0,063-0,200 mm.

Elles sont révélées soit aux UV ( 254nm ), à la ninhydrine, en vaporisant (spray léger) une solution éthanolique de ninhydrine (40 mg / 100 ml EtOH) pour révéler les amines ou les amides en chauffant à 150°C, à la fluorescamine, en vaporisant une solution (40 mg / 100 ml Acétone) pour révéler les amines primaires ou à l'iode, en recouvrant la plaque de poudre d'iode.

.Les chromatographies sur colonne ont été effectuées sur gel de silice 60 Merck de granulométrie 0,063-0,200 mm.

### d) Technique de synthèse en phase solide SPPS

La synthèse en phase solide est réalisée dans un réacteur manuel de synthèse de peptides SPPS de fabrication artisanal et l'agitateur est un Flask Shaker modele A5-6021. L'évolution de couplage des polyamines à la phase solide ainsi que l'évolution de la protection des polyamines dans la SPPS est suivie par le test de Kaiser [Kaiser, E., Colescolt, D.L., Bossinger, C.D. and Cook, P.I. *Anal. Biochem.* **34(2)***,* 595 (1970)]. La résine utilisée dans les exemples pour la SPPS est la Chlorotrityl chloride Resin de chez NOVABIOCHEM-Suisse.

### 2- MODE OPERATOIRE GENERAL

### a)-Synthèse de polyamines symétriques illustrée par la préparation de la (N,N-N',N'-Tétraaminopropyl) 1-4-diamino butane:

Dans un ballon tricol de 2 litres, on charge 147 gr de 1-4-diamino Butane et 1000 ml d'eau déminéralisée. La solution est soumise à une agitation magnétique. Par l'intermédiaire d'une ampoule isobare on charge 443 gr d'acrylonitril en 1 heure en conservant la température à 38 C°. On monte ensuite un condensateur pour reflux et on porte la masse à 80 C° avec un bain-marie durant 1 heure. Le test fluorescamine s'avère négatif et l'excès d'acrylonitril est évaporé in vacuo à 40 C°.

On obtient deux phases. La phase organique inférieure est séparée, lavée par 300 ml d'eau et transvasée dans un ballon de 1000 ml. On ajoute 170 ml d'un mélange eau/méthanol (1:1 v/v). On laisse cristalliser la nuit. Le lendemain, les cristaux sont filtrés sur verre fritté de porosité 3 de 500 ml. L

Le gâteau est lavé sur le verre fritté avec du méthanol (2x170 ml.) et de l'éther (2x150 ml.). Le produit est séché en plateau au dessicateur sous vide (26 mm) pendant une nuit. On obtient ainsi 461 gr du produit (rendement 93%). Le produit a été analysé par RMN et MS et les analyses sont compatibles. Le produit est hydrogéné sans autre purification.

Dans un autoclave inox de 1 litre on charge 30 gr du polynitrile précédent (0.1 mol). On prépare en même temps dans un becher une solution de 140 ml d'éthanol (95%) et de 8 gr de NaOH (0.2 mol). Lorsque la soude est solubilisée, on charge cette solution dans l'autoclave. De l'azote est passé dans l'autoclave et on charge 8 ml de Nickel de Raney sur charbon. L'autoclave est fermé. La pression initiale d'hydrogénation est de 52 at. et elle descend à 28.5 en 5 heures à température ambiante. La suspension est filtrée sur papier, le filtre est lavé avec de l'éthanol (2x25 ml), les filtrats sont concentrés à sec in vacuo. L'huile est mélangée avec 30 ml d'eau et extraite par 100 ml de CH₂Cl₂. La phase organique est séchée sous MgSO4, filtrée puis évaporée in vacuo. On obtient une huile fluide jaunâtre (27 gr rendement 85 %).

Le produit a été analysé par CCM (monotache), RMN et MS et les analyses étaient compatibles. Le produit est utilisé sans autre purification.

### b)- Méthode A: Ancrage de une fonction acide sur le support polymerique:

De la résine Chlorotrityl chloride (5 gr. 1.2 mmol Cl/gr. resin) est chargée dans un réacteur SPPS, 50 ml de CH₂Cl₂ sont ajoutés et le mélange est agité pendant 5 mn. De l'acide bromoacétique (1.05 gr, 7 mmol) est ajouté, suivi de DIEA (0.95 ml., 7.5 mmol). Le réacteur est agité pendant deux heures à température ambiante. Le liquide est filtré et la résine est lavée avec CH₂Cl₂ et iPrOH (10x50) et MeOH (2x50ml). Enfin, la résine est séchée sous un courant d'azote.

### c)- Méthode B : Réaction des polyamines avec la bromoacetyl-resine:

La polyamine (10 excès molaires) est dissoute dans 50 ml CH₂Cl₂ et chargée dans le réacteur contenant le produit obtenu par la méthode A. Le réacteur est agité pendant 2 hr. à température ambiante. Le solvant est filtrée et la résine lavée avec CH₂Cl₂ et iPrOH (10x50 ml), le test de Kaiser est positif.

### d)- Protection des polyamino acides sur la résine:

### Méthode C :

Les Di-tert-butyl dicarbonate (48 mmol) et DIEA (50 mmol) sont dissous dans CH₂Cl₂ (50 ml) et chargés dans le réacteur contenant le produit obtenu selon la méthode B. Le réacteur est agité pendant une nuit. Le lendemain le test de Kaiser est négatif. Le solvant est filtré et la résine est lavée alternativement avec CH₂Cl₂ et iPrOH (10x50 ml), MeOH (2x50 ml) et éther (2x50 ml). La résine est séchée sous une courant d'azote. Le test de Kaiser est toujours négatif.

### Méthode D :

La résine obtenue par le méthode B (1.5 gr) est chargée dans un ballon et CH₂Cl₂ (20 ml) est ajouté, suivi par DIEA (20 mmol). Le mélange est soumis à agitation magnétique et le chloroformate de benzyle (14 mmol) est ajouté goutte à goutte pendant 5 min. Le pH est maintenu à 11 par addition de DIEA. Après une nuit, la résine est passée dans un réacteur SPPS, filtrée et lavée alternativement avec CH₂Cl₂ et iPrOH (10x20 ml) et éther (2x20 ml). La résine est séchée sous une courant d'azote.

### e)- Méthode E : Clivage des acides polyaminées protégées de la résine:

Les résines obtenues par les méthodes C et D sont chargées dans un ballon de 250 ml équipé d'un barreau magnétique. Une solution composée de 50 ml CH₂Cl₂ et 25 ml CF₃CH₂OH est ajoutée et le mélange est agité pendant 2 hr. La solution est filtrée, la résine est lavée avec CH₂Cl₂ (2x10 ml) et les phase organiques ainsi obtenues sont rassemblées et évaporées in vacuo. Les produits sont ensuit purifiés par flash chromatographie sur SiO₂ avec comme éluant CHCl₃/MeOH (9:1). Les fractions contenant les produits sont identifiées par CCM. (Pour de plus amples détails voir les exemples çi-après.)

### f)- Méthode F : Couplage des acides aminées avec dilipidylamines:

Boc-acide amine (10 mmol) et dilipidylamine C12-C22 (10 mmol) sont chargés dans un ballon de 250 ml. CHCl₃ (100 ml) est ajouté et le mélange est agité jusqu'à dissolution complète. TEA (30 mmol) et BOP (33 mmol) sont ensuite ajoutés. Le pH est maintenu à 10 avec TEA et la réaction est agitée pendant 2 hr. Après l'achèvement de la réaction (CCM) le chloroforme est évaporé et le solide est repris dans de l'acétate d'éthyle (300 ml). La phase organique est lavée avec KHSO₄ (4x100 ml), NaHCO₃ (4x100 ml), et NaCl (4x100 ml). La phase organique est séchée sous MgSO₄ filtrée et évaporée in vacuo. Les produits sont analysés par CCM, NMR et MS et sont utilisés sans d'autres purifications. Les rendements sont de l'ordre de 90 %.

### g)- Couplage des acides polyamines protégés avec dilipidyl acides amides et clivage des groupements de protection Boc et Z

### Méthode G

Le produit obtenue par le méthode F (9 mmol) est chargé dans un ballon équipé d'un barreau magnétique et du TFA froid (4 C°) est ajouté (30 ml). La solution est agitée pendant 1 hr. Le TFA est évaporé in vacuo. Le produit est dissous par l'addition de DMF (70 ml). On ajoute du TEA (30 mmol) puis de l'acide polyamine protégé obtenu par la méthode E (9 mmol). Le pH est ajusté à 10 et BOP (33 mmol) est ajouté. La solution est agitée pendant 2 hr. et suivie par CCM. Après l'achèvement du couplage (CCM), une solution de KHSO₄ est ajoutée (700 ml) et le produit est extrait avec acétate d'éthyle (3x100 ml). La phase organique est lavée avec KHSO₄ (3x50), NaHCO₄ (3x50) et NaCl (3x50 ml), séchée sous MgSO₄, filtrée et évaporé in vacuo. Les produits sont analysés par RMN, CCM et MS et sont déprotégés sans purification préalable. Du TFA (50 ml) est ajouté au produit et la solution est agitée pendant 1.5 hr., le TFA est évaporé. Si le produit contient encore de groupements Z ou CIZ qui sont pas clivables au TFA le méthode H est suivie directement. Les produits finaux sont purifiés par CLHP semipréparative( voir exemples).

### Méthode H

Les produits obtenus par le méthode G contenant des groupe Z ou CIZ sont chargés dans un ballon équipé d'un barreau magnétique et dissous dans 10 ml MeOH/gr produit. Les Pd/C (10 %, 1gr/Gr produit) et formiate d'ammonium(1 gr/gr produit) sont ajoutés à température ambiante. L'hydrogénation est suive par CLHP. Après 2 hr la réaction est finie, le mélange est filtré et le filtre lavé avec 10 ml MeOH. De l'eau double distillée est ajoutée et la solution est congelée et lyophilisée. Les produits finaux sont purifiés par CLHP préparative.

### h) Méthode I de déprotection des groupements de protection Boc

L'acide trifluoro acétique (50 ml) est ajouté au produit contenant des groupements Boc (1 mmol) dans un ballon. La solution est agitée pendant 1.5 hr, le TFA est évaporé. L'amine est complètement deprotégée et prête à l'emploi pour des couplages sans autre purification.

### B-MATERIEL ET METHODE POUR L'ETUDE BIOLOGIOUE:

### 1 PLASMIDES UTILISES POUR LE TRANSFERT DE GENES IN VITRO

Le plasmide pCMV-LUC est une construction dérivée, soit du plasmide pGL2-Basic Vector (Promega), soit du plasmide pGL2-Control Vector (Promega) par insertion d'un fragment Mlu I-Hind III contenant le promoteur du Cytomegalovirus humain (CMV) extrait du plasmide vecteur pcDNA3 (Invitrogen).

### 2 PROTOCOLE DE PREPARATION DES SOLUTIONS UTILISEES POUR LA TRANSFECTION

Les produits décrits dans l'invention sont mis en solution à 20 mM dans l'éthanol ou dans l'eau ,puis dilués dans l'eau en s'assurant que la concentration éthanolique finale est inférieure à 10%.

Les solutions d'acide nucléique diluées en sérum physiologique (NaCl 0,15M) sont ajoutées aux solutions de lipofectant dans un rapport 1/1 (v/v). Après homogénéisation au vortex et incubation 15 minutes à température ambiante les solutions ADN/lipofectant sont distribuées à 9% (v/v) final dans les puits où les cellules ont été lavées par du milieu de croissance dépourvu de protéines (sérum) et remises en croissance en milieu dépourvu ou non de sérum.

### C MATERIEL POUR LES ESSAIS IN VIVO

### 1 MATÉRIELS

### a) Modèles expérimentaux:

- souris C57/BL 6 adultes (>8 semaines) femelles
- tumeurs de type 3LL (Lewis Lung carcinoma) obtenues par passage de fragments de tumeur d'animal à animal, implantés sous la peau au niveau du flanc.

### b) Plasmides utilisés:

pXL 2622: il dérive du pGL2 basic, (Promega) dans lequel le promoteur du cytomegalovirus (CMV) extrait de pCDNA3 (Invitrogen) a été inséré en amont du gène codant pour la luciférase. Ce plasmide est obtenu par le technique de précipitation au PEG (Ausubel), et stocké dans du Tris 10mM EDTA 1mM pH 8 à 4 °C à une concentration d'environ 10 µg d'ADN par µl.

### 2 PROTOCOLES

Solutions injectées: L'ADN à transfecter est d'abord solubilisé dans le tampon, le peptide (KTPKKAKKP)₂ SEQ ID N°1 est alors ajouté, et après 20 minutes une solution de lipides cationiques à forte concentration (20 ou 40 mM) est ajoutée au mélange. Après addition de tous les produits, le mélange contient, outre l'ADN ( en concentration finale de 0,5mg/ml), le peptide (0,75mg/ml) et le lipide cationique, NaCl 150mM, D-Glucose 5% et MES 5mM pH 6,2. L'injection est réalisée 20 à 30 minutes après la préparation de la solution.

### EXEMPLE 1:

### Synthèse de H₂N(CH₂)₃NH(CH₂)₄NH(CH₂)₃NHCH₂COGlyN[(CH₂)_{17CH3}]₂ (6)

### a- Synthèse de l'acide {Boc-[3-(Boc-{4-[Boc-(3-Boc-amino-propyl)-amino]-butyl}amino)propyl]-amino}-acétique (3)

La résine obtenue selon la méthode A est mise à réagir avec la spermine selon les méthodes B, C et E. Le produit protégé est purifie par chromatographie sur SiO₂. Le rendement est de 40 %.
CCM: R_{f}= 0.32 (CHCl₃/MeOH, 9:1)
CLHP, Rₜ= 4,22 min, (H₂O/MeCN: 3 min [40/60], 3-20 min [0/100], 35 min [0/100] Spectre de R M N ¹H (400 MHz, (CD₃)₂SO d6 avec ajout de quelques gouttes de CD₃COOD d4, d en ppm) : 1,40 (4 s, 36H : C(CH₃)₃); 1,46 (mt, 4H : CH₂CH₂ centraux du butyle) ; 1,64 et 1,74 (2 mts, 2H chacun : CH₂ central des propyles) ; 2,96 (t, J = 7 Hz, 2H : CH₂NCOO) ; 3,15 (mt, 8H : CH₂NCH₂) ; 3,23 (t, J = 7,5 Hz, 2H : CH₂NCOO) ; 3,83 (s, 2H : OCONCH₂COO).
MH⁺: 661

### b- Synthèse de l'acide {Z-[3-(Z-{4-[Z-(3-Z-amino-propyl)-amino]-butyl}amino) propyl]-amino}-acétique (4)

La résine obtenue selon la méthode A est mise à réagir avec de la spermine selon les méthodes B, C et D. Le produit protégé est purifié par chromatographie sur SiO₂. Le rendement est de l'ordre de 20 %.
CCM: R_{f}= 0,85 (CHCl₃/MeOH, 8:2)
CLHP, Rₜ= 6.92 min, (H₂O/MeCN: 3 min [40/60], 3-20 min [0/100], 35 min [0/100]
Spectre de R M N ¹ H (400 MHz, (CD₃)₂SO d6, à une température de 413K, d en ppm) : 1,49 (mt, 4H : CH₂CH₂ centraux du butyle) ; 1,74 et 1,81 (2 mts, 2H chacun : CH₂ central des propyles) ; 3,07 (q, J = 7 Hz, 2H : CH₂NCOObenzyle) ; de 3,15 à 3,30 (mt, 8H : CH₂NCH₂) ; 3,33 (t, J = 7,5 Hz, 2H : NCH₂COO) ; 3,70 (s, 2H : OCONCH₂COO) ; 5,07 - 5,10 - 5,12 et 5,13 (4 s, 2H chacun : ArCH₂OCON) ; 6,65 (mf, 1H : NHCO) ; de 7,25 à 7,40 (mt, 20H : H aromatiques).
MH⁺: 797

### c- Boc-Gly-dioctadecylamide. (5)

Le Boc-Gly est couplé à la dioctadécylamine selon la méthode F, rendement de 90 %
CCM: R_{f} = 0.9 (CHCl₃/MeOH, 9:1)
MH⁺= 679
Spectre de R M N ¹ H (300 MHz, CDCl₃, d en ppm) : 0,89 (t, J = 7 Hz, 6H : CH₃) ; 1,29 (mt, 60H : CH₂ centraux des chaînes grasses) ; 1,49 (s, 9H : C(CH₃)₃); 1,55 (mt, 4H : 1 CH₂ de chaque chaîne grasse) ; 3,15 et 3,33 (2 t, J = 7,5 Hz, 2H chacun : NCH₂ des chaînes grasses) ; 3,95 (d,J = 5 Hz, 2H : OCONCH₂CON) ; 5,57 (mf, 1H : CONH).

### d- H₂N(CH₂)₃NH(CH₂)₄NH(CH₂)₃NHCH₂COGlyN[(CH₂)₁₈]₂ (6)

Les produits (3) et (5) ou (4) et (5) sont couplés selon la méthode G. Les produits sont déprotégés comme décrit dans la méthode G pour le produit protégé Boc et méthode H pour le produit protégé Z. Le produit est purifié par CLHP semipréparative et les fractions analysées par CLHP.
CLHP, Rₜ= 15.35 min, (H₂O/MeCN: 3 min [40/60], 3-20 min [0/100], 35 min [0/100]
BYK 2 053Spectre de R M N ¹ H (400 MHz, (CD₃)₂SO d6 avec ajout de quelques gouttes de CD₃COOD d4, à une température de 300K, d en ppm) : 0,83 (t, J = 7 Hz, 6H : CH₃) ; 1,23 (mt, 60H : CH₂ centraux des chaînes grasses) 1,43 et 1,53 (2 mts, 2H chacun : 1 CH₂ de chaque chaîne grasse) ; 1,63 (mt, 4H : CH₂CH₂ centraux du butyle) ; 1,96 (mt, 4H : CH₂ central des propyles) ; 2,93 - 3,00 et 3,22 (3 mts, 16H en totalité : NCH₂) ; 3,83 (s, 2H : NCH₂CON) ; 4,03 (s, 2H : CONCH₂CON).
MH⁺= 821

### EXEMPLE 2:

### Synthèse de H₂N(CH₂)₃NH(CH₂)₄NH(CH₂)₃NHCH₂CON[(CH₂)₁₈]₂ (7)

Le produit (3) est couplé avec la dioctadécylamine selon la méthode F et déprotégé selon la méthode G. Le produit est purifié par CLHP semipréparative et les fractions analysées par CLHP.
CLHP, Rₜ= 15.2 min, (H₂O/MeCN: 3 min [40/60], 3-20 min [0/100], 35 min [0/100]
Spectre de R M N ¹ H (400 MHz, dans un mélange 2/3 de CF₃COOD et 1/3 de CD₃COOD d4, d en ppm) : 0,78 (t, J = 7 Hz, 6H : CH₃) ; 1,20 (mt, 60H : CH₂ centraux des chaînes grasses) ; 1,52 (mt, 4H : 1 CH₂ de chaque chaîne grasse) ; 1,80 (mt, 4H : CH₂CH₂ centraux du butyle) ; 2,23 et 2,32 (2 mts, 2H chacun : CH₂ central des propyles) ; de 3,10 à 3,40 (3 mts, 16H en totalité : NCH₂) ; 4,15 (s, 2H : NCH₂CON).
MH⁺= 764

### EXEMPLE 3:

### Synthèse de H₂N(CH₂)₃NH(CH₂)₄NH(CH₂)₃NHCH₂COArgN[(CH₂)₁₈]₂ (9)

### a- Boc-Arg(Z₂)dioctadecylamide. (8)

Le produit est synthétisé par couplage de BocArg(Z₂) et de dioctadécylamine par la méthode F avec un rendement de 91 %
CCM Rf= 0.9 (CHCl3/MeOH, 9:1)
MH⁺=1046

### b- H₂N(CH₂)₃NH(CH₂)₄NH(CH₂)₃NHCH₂COArgN[(CH₂)₁₈]₂ (9)

Le produit (3) ou (4) est couplé au produit (8) par la méthode G et déprotégé par la méthode G (Boc) et/ou H (Z). Le produit final est purifié par CLHP semipréparative et les fractions analysées par CLHP analytique.
CLHP, Rₜ= 13.83 min, (H₂O/MeCN: 3 min [40/60], 3-20 min [0/100], 35 min [0/100]
Spectre de R M N ¹ H (400 MHz, (CD₃)₂SO d6, δ en ppm) : 0,90 (t, J = 7 Hz, 6H : CH₃) ; 1,28 (mt, 60H : CH₂ des chaines grasses) ; de 1,40 à 1,80 (mt, 12H : CH₂) ; 1,93 (mt, 4H : CH₂ central des propyles) ; de 2,80 à 3,10 (mt, 16H : NCH₂ et NCH₂ des chaines grasses) ; 3,42 (mt, 2H : CH₂N de l'amido) ; 3,77 (mt, 2H :NCH₂CON); 4,67 (mt, 1H : NCHCON) : de 6,80 à 7,50 (mf étalé, 2H : NH₂) ; 7,78 - 7,92 - 8,80 et 9,03 (respectivement mt et 3mfs, respectivement 1H - 2H - 4H et 1H : CONH - NH et NH₂).
MH⁺: 920

### EXEMPLE 4:

### Synthèse de H₂N(CH₂)₃NH(CH₂)₄NH(CH₂)₃NHCH₂COArg(Z)₂N[(CH₂)₁₇-CH₃]₂ (10)

Le produit (3) est couplé au produit (8) par la méthode G et les groupes Boc clivés par la même méthode. Le produit est purifié par CLHP semipréparative et les fractions analysées par CLHP analytique
CLHP, Rₜ = 17.75 min, (H₂O/MeCN: 3 min [40/60], 3-20 min [0/100], 35 min [0/100]
Spectre de R M N ¹ H (400 MHz, (CD₃)₂SO, d en ppm) : 0,87 (t, J = 7 Hz, 6H : CH₃) ; 1,25 (mt, 60H : CH₂ centraux des chaînes grasses) ; 1,40 et 1,57 (2 mts, 2H chacun : 1 CH₂ de chaque chaîne grasse) ; 1,65 (mt, 8H : CH₂CH₂ centraux des butyles) ; 1,95 (mt, 4H : CH₂ central des propyles) ; de 2,85 à 3,05 (mt, 14H en totalité : NCH₂) ; 3,23 (t, J = 7,5 Hz, 2H : NCH₂) ; 3,75 (s, 2H : NCH₂CON) ; 3,85 et 3,95 (2 mts, 1H chacun : CH₂NC) ; 4,67 (mt, 1H : CONCHCON) ; 5,07 et 5,25 (respectivement AB limite et s, J = 13,5 Hz, 2H chacun : NCOOCH₂Ar) ; de 7,25 à 7,45 (mt, 10H : H aromatiques) ; 7,95 - 8,85 - 9,00 et 9,20 (4 mfs : H échangeables).
MH⁺:1188

### EXEMPLE 5:

### Synthèse de H₂N(CH₂)₃NH(CH₂)₄NH(CH₂)₃NHCH₂COLys(rhodamine)N[(CH₂)₁₇-CH₃]₂ (13)

### a- Boc-Lys(Z)dioctadecylamide (11)

Le produit a été synthétisé par couplage de BocLys(ClZ) avec le dioctadécylamine par la méthode F avec un rendement de 89 %.
CCM, Rf= ,92 (CHCl3/MeOH, 9:1)
MH⁺: 918

### b-BocHN(CH₂)₃NBoc(CH₂)₄NBoc(CH₂)₃NBocCH₂COLys(CIZ)N[(CH₂)₁₇-CH₃]₂ (12)

Le produit (3) est couplé au produit (11) par la méthode G (sans la déprotection du Boc).
Spectre de R M N ¹ H (400 MHz, (CD₃)₂SO d6, à une température de 423 K, d en ppm) : 0,92 (t, J = 6,5 Hz, 6H : CH₃) ; 1,32 (mt, 60H : CH₂ centraux des chaînes grasses) ; 1,44 (2 s, 36H en totalité : C (CH₃)₃) ; de 1,50 à 1,80 (mt, 16H : 1 CH₂ de chaque chaîne grasse - CH₂CH₂ centraux du butyle - CH₂CH₂CH₂ et CH₂ central du propyle) ; 3,00 (q, J = 6,5 Hz, 2H : OCONCH₂) ; 3,05 (q, J = 6,5 Hz, 2H : CH₂NCOO) ; de 3,15 à 3,40 (mt, 14H : NCH₂ des chaînes grasses - CH₂NCH₂ et CH₂NCH₂CH₂N) ; 3,80 (s, 2H : OCONCH₂CON) ; 4,75 (mt, 1H : CONCHCON) ; 5,15 (s, 2H : NCOOCH₂Ar) ; 5,97 et 6,53 (2 mts, 1H chacun : OCONH et NHCOO) ; 7,08 (d, J = 7,5 Hz , 1H : CONH) ; de 7,30 à 7,50 (mts , 4H : H aromatiques).

### c-H₂N(CH₂)₃NH(CH₂)₄NH(CH₂)₃NHCH₂COLys(rhodamine)N[(CH₂)₁₇-CH₃]₂ (13)

Le groupement CIZ sur la lysine du produit (12) est clivé par la méthode H, le produit ainsi obtenu est séché in vacuo et repris dans de l'éther et rincé avec NaHCO3 et NaCl. L'éther est séché sur de MgSO4 et évaporé in vacuo.

77 mg (60 µm) du produit déprotégé sont dissous dans 3 ml de MeOH, le DIEA (64 µL ) est ajouté puis la tétramethyl rhodamine isothiocyanate (30 mg, 68 µm); la solution est agitée pendant 17 hr et la reaction est suivie par CCM. Le lendemain la solution est concentrée à sec in vacuo. Le TFA (4ml) est ensuite ajouté et on laisse agiter pendant 1 hr. Le TFA est évaporé et le produit brut est purifié par CCLHP semipréparative avec un rendement final de 30 %.
CCM. Rf= 0.05 (MeOH)
CLHP (semiprep.) Rₜ= 61,55 min. (H2O/MeCN: 3 min [100/0], 3-45 min [0/100], 45-140 min. [0/100].
MH⁺:1335

### EXEMPLE 6:

### Synthèse de H₂N(CH₂)₃NH(CH₂)₄NH(CH₂)₃NHCH₂COLys(biotinyl)N[(CH₂)₁₇-CH₃]₂ (14)

Le produit (12) est déprotégé par la méthode H (271 mg, 0,21 mmol) et dissous dans du DMF (10 ml), le DIEA (0,11ml) est ajouté puis la biotine (56,4 mg, 0,23 mmol) et le BOP (102 mg, 0,23 mmol; le pH est maintenu à 10 (DIEA) et la fin de la réaction est vérifiée par le test fluorescamine. Le produit est récupéré comme décrit dans la méthode F et déprotégé sans autre purification avec TFA (5 ml) pendant 1 hr. Le TFA est évaporé et le produit purifié par CHLP semipréparative avec un rendement de 50 %.
CLHP, Rₜ= 13.12 min, (H₂O/MeCN: 3 min [40/60], 3-20 min [0/100], 35 min [0/100]
MH⁺:1118

### EXEMPLE 7

### Synthèse de {H₂N(CH₂)₃}₂N(CH₂)₄N{(CH₂)₃NH₂}(CH₂)₃NHCH₂COGlyN[(CH₂)₁₇-CH₃]₂ (16)

### a- {BocNH(CH₂)₃}₂N(CH₂)₄N{(CH₂)₃NHBoc}(CH₂)₃NBocCH₂COOH (15)

Le produit (1) est ancré sur le polymère par la méthode B, protégé par la méthode C et clivé de la résine par la méthode E. Le produit est purifié sur SiO2 avec un rendement de 35 %.
CCM: R_{f}= 0,2 (CHCl₃/MeOH, 8:2)
Spectre de R M N ¹ H (400 MHz, (CD₃)₂SO d6 avec ajout de quelques gouttes de CD₃COOD d4, à une température de 433K, d en ppm) : 1,42 (s, 36H : C(CH₃)₃) ; 1,56 (mt, 4H : CH₂CH₂ centraux du butyle) ; de 1,65 et 1,85 (mt, 8H : CH₂ central des propyles) ; 2,76 (mt, 12H : CH₂N(CH₂)₂) ; 3,06 (t, J = 6,5 Hz, 6H : OCONCH₂) ; 3,29 (mt, 2H : NCH₂) ; 3,86 (s, 2H : OCONCH₂COO).
MH⁺= 775

### b-{H₂N(CH₂)₃}₂N(CH₂)₄N{(CH₂)₃NH₂}(CH₂)₃NHCH₂COGlyN[(CH₂)₁₇-CH₃]₂(16)

Le produit (15) est couplé avec le produit (5) selon la méthode G. Le produit est déprotégé par méthode G et est purifié sur CLHP semipréparative, les fractions sont analysées par CLHP analytique et lyophilisées.Rendement de 55 %.
CLHP (semiprep.): Rₜ= 38,72 min (H₂O/MeCN, 10 min[100/0], 10-45 min [0/100], 45-140 min [0/100]
Spectre de R M N ¹ H (400 MHz, (CD₃)₂SO d6, à une température de 386 K, d en ppm) : 0,90 (t, J = 7 Hz, 6H : CH₃) ; 1,30 (mt, 60H : CH₂ centraux des chaînes grasses) ; 1,55 (mt, 4H : 1 CH₂ de chaque chaîne grasse) ; 1,65 (mt, 4H : CH₂CH₂ centraux du butyle) ; 1,97 (mt, 8H : CH₂ central des propyles) ; de 2,80 à 3,05 - 3,06 et 3,28 (respectivement mt et 2 t, J = 7,5 Hz, 18H - 2H et 4H : NCH₂) ; 3,80 (s, 2H : NCH₂CON) ; 4,03 (d, J = 5,5 Hz, 2H : CONCH₂CON) ; de 6,00 à 9,00 (mf étalé : NH₂ et NH) ; 8,27 (mt, 1H : CONH).
MH⁺: 935

### EXEMPLE 8

### Synthèse de: {H₂N(CH₂)₃}₂N(CH₂)₄N{(CH₂)₃NH₂}(CH₂)₃NHCH₂CON[(CH₂)₁₇-CH₃]₂ (17)

Il est synthétisé comme le produit (7)en utilisant le produit (15) à la place du (3). Le produit est purifié par CLHP semipréparative et les fractions analysées par CLHP analytique et lyophilisées.
CLHP (semiprep.): Rₜ= 38 min (H₂O/MeCN, 10 min[100/0], 10-45 min [0/100], 45-140 min [0/100]
Spectre de R M N ¹ H (400 MHz, (CD₃)₂SO d6 avec ajout de quelques gouttes de CD₃COOD d4, d en ppm) : 0,88 (t, J = 7 Hz, 6H : CH₃) ; 1,29 (mt, 60H : CH₂ centraux des chaînes grasses) ; 1,52 (mt, 4H : 1 CH₂ de chaque chaîne grasse) ; 1,68 (mt, 4H : CH₂CH₂ centraux du butyle) ; de 1,90 à 2,10 (mt, 8H : CH₂ central des propyles) ; 2,90 - de 2,95 à 3,15 -3,18 et 3,15 (respectivement t - mt et 2 t larges, J = 7,5 Hz, 24H en totalité : NCH₂) ; 4,02 (s, 2H : NCH₂CON).
MH⁺= 878

### EXEMPLE 9

### Synthèse de {H₂N(CH₂)₂}₂N(CH₂)₂NHCH₂COGlyN[(CH₂)₁₇-CH₃]₂ (19)

### a- {BocNH(CH₂)₂}₂N(CH₂)₂NBocCH₂COOH (18)

Il est synthétisé comme le produit (15) en utilisant la tris(aminoehyl)amine à la place du produit (1). Rendement 29 %.
CCM, R_{f}= 0,55 (CHCl₃/MeOH, 8:2)
Spectre de R M N ¹ H (400 MHz, (CD₃)₂SO d6 à une température de 393 K, d en ppm) : 1,44 (s, 27H : C(CH₃)₃); 2,58 (t, J = 6,5 Hz, 4H : CH₂NCH₂) ; 2,66 (t, J = 7 Hz, 2H : NCH₂) ; 3,04 (q, J = 6,5 Hz, 4H : OCONCH₂) ; 3,28 (t, J = 7 Hz, 2H : OCONCH₂) ; 3,76 (s, 2H : OCONCH₂COO) ; 6,06 (mf, 2H : CONH).
MH⁺= 505

### b- {H₂N(CH₂)₂}₂N(CH₂)₂NHCH₂COGlyN[(CH₂)₁₇-CH₃]₂ (19)

Il est synthétisé comme le produit (17) en utilisant (18) à la place de (15) avec un rendement de 65%.
CLHP. Rₜ= 122 min , (H₂O/MeCN, 10 min[100/0], 10-45 min [0/100], 45-140 min [0/100]
Spectre de R M N ¹ H (400 MHz, (CD₃)₂SO d6 avec ajout de quelques gouttes de CD₃COOD d4, d en ppm) : 0,87 (t, J = 7 Hz, 6H : CH₃) ; de 1,15 à 1,35 (mt, 60H : CH₂ centraux des chaînes grasses) ; 1,45 et 1,55 (2 mts, 2H chacun : 1 CH₂ de chaque chaîne grasse) ; 2,64 (t, J = 5,5 Hz, 4H : CH₂NCH₂) ; 2,75 (t, J = 6 Hz, 2H : NCH₂) ; 2,95 (t, J = 5,5 Hz, 4H : NCH₂) ; 3,08 (t, J = 6 Hz, 2H : NCH₂) ; 3,25 (mt, 4H : NCH₂ des chaînes grasses) ; 3,88 (s, 2H : NCH₂CON) ; 4,06 (d, J = 5 Hz, 2H : CONCH₂CON) ; 7,75 (mf étalé résiduel : NH) ; 8,68 (t résiduel, J = 5 Hz : CONH).
MH+= 765

### EXEMPLE 10

### Synthèse de {H₂N(CH₂)₂}₂N(CH₂)₂NHCH₂CON[(CH₂)₁₇-CH₃]₂ (20)

Il est synthétisé comme le produit (19) en utilisant le dioctadécylamine à la place de (5). Rendement de 73 %.
CLHP, Rₜ= 100,1 min, (H₂O/MeCN, 10 min[100/0], 10-45 min [0/100], 45-140 min [0/100]
MH⁺= 708

### EXEMPLE 11

### Synthèse de NH₂(CH₂)₃NH(CH₂)₄NH(CH₂)₃NHCH₂COLysN[(CH₂)₁₇CH₃]₂ (21) (RPR 127888 A )

Le produit (12) est deprotegé par la méthode H (Cl-Z), suivie de la méthode I. Le produit final est purifié par CLHP semipréparative et les fractions analysées par CLHP analytique.
CLHP, Rt= 11.76 min,(H₂O/MeCN: 3 min [60/40], 3-20 min [0/100], 35 min [0/100] MH⁺:892
Spectre de R M N ¹H (400 MHz, (CD₃)₂SO d6, à une température de 393K, d en ppm) : 0,91 (t, J = 7 Hz, 6H : CH₃ des chaînes grasses) ; 1,31 (mt, 60H : (CH₂)₁₅ centraux des chaînes grasses) ; de 1,35 à 1,75 (mt, 10H : 1 CH₂ de chaque chaîne grasse, (CH₂)₃ centraux du lysyle) ; 1.75 (mt, 4H : (CH₂)₂ centraux du butyle) ; 2,00 (mt, 4H : CH2 des propyles) ; 2,82 - 2,98 - 3,06 et de 3,10 à 3,50 (respectivement 2 t - mt et 2mfs, J = 7 Hz, 18H en totalité : NCH₂ du lysyle - NCH₂ du butyle - NCH₂ des propyles et NCH₂ des chaînes grasses) ; 3,62 (s, 2H : NCH₂CON) ; 4,73 (q, J = 7 Hz, 1H : CONCHCON du lysyle) ; 8,18 (d, J = 7 Hz, 1H : CONH du lysyle).

### EXEMPLE 12

### Synthèse de NH₂(CH₂)₃NH(CH₂)₄NH(CH₂)₃NHCH₂COLys(CI-Z)N[(CH₂)₁₇CH₃]₂ (22) (RPR 122759 A)

Le produit (12) est déprotégé par la méthode I. Le produit final est purifié par CLHP semipréparative et les fractions analysées par CLHP analytique.
CLHP, Rt= 16.79 min,(H₂O/MeCN: 3 min [60/40], 3-20 min [0/100], 35 min [0/100] MH⁺:1060
Spectre de R M N ¹H (400 MHz, (CD₃)₂SO d6, à une température de 373K, d en ppm) : 0,91 (t, J = 7 Hz, 6H : CH₃ des chaînes grasses) ; 1,31 (mt, 60H : (CH₂)₁₅ centraux des chaînes grasses) ; de 1,30 à 1,75 (mt, 10H : 1 CH₂ de chaque chaîne grasse, (CH₂)₃ centraux du lysyle) ; 1.72 (mt, 4H : (CH₂)₂ centraux du butyle) ; 1,95 (mt, 4H : CH₂ des propyles) ; 2,98 - 3,06 et de 2.90 à 3,50 (respectivement 2 mts et mf, 18H en totalité : NCH₂ du lysyle - NCH₂ du butyle - NCH₂ des propyles et NCH₂ des chaînes grasses) ; 3,59 (s, 2H : NCH₂CON) ; 4,75 (q, J = 7 Hz, 1H : CONCHCON du lysyle) ; 5,16 (s, 2H : COOCH₂Ar) ; 6,85 (mf, 1H : OCONH) ; de 7,35 à 7,55 (mt, 5H : H Aromatiques) ; 8,15 (mf, 1H : CONH du lysyle).

### EXEMPLE 13

### Synthèse de NH₂(CH₂)₃NH(CH₂)₄NH(CH₂)₃NHCH₂COLys(CHO)N[(CH₂)₁₇CH₃]₂ (24) (RPR 122760 A) (24).

### a- NHBoc(CH₂)₃NBoc(CH₂)₄NBoc(CH₂)₃NBocCH₂COLysN[(CH₂)₁₇CH₃]₂ (23)

Le produit (12) est déprotégé par la méthode H (Cl-Z) avec rendement de 65% et utilisé sans autre purification.
CLHP, Rt= 20.82 min,(H₂O/MeCN: 3 min [60/40], 3-20 min [0/100], 35 min [0/100]

### b- NH₂(CH₂)₃NH(CH₂)₄NH(CH₂)₃NHCH₂COLys(CHO)N[(CH₂)₁₇CH₃]₂ (24)

Le produit (23) est couplé avec l'acide formique par la méthode G. Le produit est purifié par CLHP semipréparative et les fractions analysées par CLHP analytique.
CLHP, Rt=13.60 min,(H₂O/MeCN: 3 min [60/40], 3-20 min [0/100], 35 min [0/100]
MH⁺: 920
Spectre de R M N ¹H (400 MHz, (CD₃)₂SO d6, à une température de 383K, d en ppm) : 0,92 (t, J = 7 Hz, 6H : CH₃ des chaînes grasses) ; 1,31 (mt, 60H : (CH₂)₁₅ centraux des chaînes grasses) ; de 1,35 à 1,70 (mt, 10H : 1 CH₂ de chaque chaîne grasse, (CH₂)₃ centraux du lysyle) ; 1.73 (mt, 4H : (CH₂)₂ centraux du butyle) ; 1,98 (mt, 4H : CH₂ des propyles) ; de 2.85 à 3,50 (mt, 18H : NCH₂ du lysyle - NCH2 du butyle - NCH₂ des propyles et NCH₂ des chaînes grasses) ; 3,62 (s, 2H : NCH₂CON) ; 4,75 (mt, 1H : CONCHCON du lysyle) ; 7,60 (mf, 1H : CONH) ; 8,05 (s large, 1H : CH de l'aldéhyde) ; 8,18 (mf, 1H : CONH du lysyle).

### EXEMPLE 14

### Synthèse de NH₂(CH₂)₃NH(CH₂)₄NH(CH₂)₃NHCH₂COLys[Cholesteryl]N[(CH₂)₁₇CH₃]₂ (25) (RPR 128142 A)

Le produit (23) est couplé avec du cholesteryl chloroformate selon la méthode G (sans utilisation du réactif BOP). Le produit est purifié par CLHP semipréparative et les fractions analysées par CLHP analytique.
CLHP, Rt=21.66 min,(H₂O/MeCN: 3 min [60/40], 3-20 min [0/100], 35 min [0/100] MH⁺: 1304
Spectre de R M N ¹H (600 MHz, (CD₃)₂SO d6, d en ppm) : 0,68 et 0,98 (2 s, 3H chacun : CH₃ en 18 et CH₃ en 19 du cholestéryle) ; 0,86 (mt, 12H : CH₃ des chaînes grasses et CH₃ en 26 et 27 du cholestéryle) ; 0,91 (d, J = 7 Hz, 3H : CH₃ en 21 du cholestéryle) ; 1,31 (mt, 60H : (CH₂)₁₅ centraux des chaînes grasses) ; de 0,80 à 2,30 (mt, 42H : 1 CH₂ de chaque chaîne grasse - CH₂ en 1, 2, 4, 7, 11, 12, 15, 16, 22, 23 et 24 du cholestéryle - CH en 8, 9, 14, 17, 20 et 25 du cholestéryle - (CH₂)₃ centraux du lysyle et CH₂ des propyles) ; 1.65 (mt, 4H : (CH₂)₂ centraux du butyle) ; 2,88 et 2,96 (2 mts, 14H en totalité : NCH₂ du lysyle - NCH₂ du butyle - NCH₂ des propyles) ; de 3,20 à 3,50 (mt, 4H : NCH₂ des chaînes grasses) ; 3,64 (s, 2H : NCH₂CON) ; 4,23 (mt, 1H: CH en 3 du cholestéryle) ; 4,63 (mt, 1H : CONCHCON du lysyle) ; 5,30 (mt, 1H : CH en 6 du cholestéryle) ; 6,98 (mt, 1H : NHCOO) ; 7,90 (mt, 1H : CONH du lysyle) ; 8,60 à 9,10 (mfs échangeables).

### EXEMPLE 15

### Synthèse de NH₂(CH₂)₃NH(CH₂)₄NH(CH₂)₃NHCH₂COLys[Arachidonyl]N[(CH₂)₁₇CH₃]₂ (26) (RPR 130605)

Le produit (23) est couplé avec de l'acide arachidonique sous courant d'azote et à l'abris de la lumière selon la méthode G. Le produit est purifié par CLHP semipréparative et les fractions analysées par CLHP analytique.
CLHP, Rt=20.67 min,(H₂O/MeCN: 3 min [60/40], 3-20 min [0/100], 35 min [0/100]
MH⁺: 1177
Spectre de R M N ¹H (400 MHz, (CD₃)₂SO d6 avec ajout de quelques gouttes de CD₃COOD d4, à une température de 393K, d en ppm) : 0,90 (t, J = 7 Hz, 6H : CH₃ des chaînes grasses) ; 0,91 (t, J = 7 Hz, 3H : CH₃ de l'arachidonyle) ; 1,31 (mt, 60H : (CH₂)₁₅ centraux des chaînes grasses) ; de 1,35 à 1,75 (mt, 18H : 1 CH₂ de chaque chaîne grasse - (CH₂)₃ centraux et CH₂ central de l'arachidonyle et (CH₂)₃ centraux du lysyle) ; 1.75 (mt, 4H : (CH₂)₂ centraux du butyle) ; 2,02 (mt, 4H : CH₂ des propyles) ; 2,10 (mt, 6H : COCH₂ et les deux =CCH₂ de l'arachidonyle) ; 2,80 - 2,97-3,06 et de 3,10 à 3,50 (respectivement mt - t - mt et 2 mfs, J = 7 Hz, 24H en totalité : =CCH₂C= de l'arachidonyle - NCH₂ du lysyle - NCH₂ du butyle - NCH₂ des propyles et NCH₂ des chaînes grasses) ; 3,62 (s, 2H : NCH₂CON) ; 4,73 (dd, J = 8 et 5 Hz, 1H : CONCHCON du lysyle) ; 5,38 (mt, 8H : CH=CH de l'arachidonyle).

### EXEMPLE 16

### Synthèse de NH₂(CH₂)₃NH(CH₂)₄NH(CH₂)₃NHCH₂COGluN[(CH₂)₁₇CH₃]₂ (28) (RPR 126097 A )

### a- Boc-Glu(O-Bz)-dioctadécylamine (27)

Le produit est synthétisé par couplage de Boc- GLU(OBz) et de dioctadécylamine par la méthode F avec un rendement de 90 %
CCM Rf= 0.88 (CHCl₃/MeOH, 9:1)

### b- NH₂(CH₂)₃NH(CH₂)₄NH(CH₂)₃NHCH₂COGluN[(CH₂)₁₇CH₃]₂ (28)

Le produit (3) ou (4) est couplé au produit (27) par la méthode G, suivie de la méthode H (déprotection Cl-Z). Le produit final est purifié par CLHP semipréparative et les fractions analysées par CLHP analytique.
CLHP, Rt= 14.64 min,(H₂O/MeCN: 3 min [60/40], 3-20 min [0/100], 35 min [0/100]
MH⁺: 893
Spectre de R M N ¹H (400 MHz, (CD₃)₂SO d6, à une température de 383K, d en ppm) : 0,90 (t, J = 7 Hz, 6H : CH₃ des chaînes grasses) ; 1,30 (mt, 60H : (CH₂)₁₅ centraux des chaînes grasses) ; 1,56 (mf, 4H : 1 CH₂ de chaque chaîne grasse) ; de 1,60 à 2,00 (mt, 2H : CH₂ central du glutaryle) ; 1.73 (mt, 4H : (CH₂)₂ centraux du butyle) ; 1,98 (mt, 4H : CH₂ des propyles) ; 2,32 (t, J = 7 Hz, 2H : COCH₂ du glutaryle) ; 3,00 - 3,06 et 3,45 (respectivement t et 2 mts, J = 7 Hz, 16H en totalité : NCH₂ du butyle - NCH₂ des propyles et NCH₂ des chaînes grasses) ; 3,65 (s large, 2H : NCH₂CON) ; 4,85 (mt, 1H : CONCHCON du glutaryle) ; 8,19 (s large, 1H: CONH du glutaryle).

### EXEMPLE 17

### Synthèse de NH₂(CH₂)₃NH(CH₂)₄NH(CH₂)₃NHCH₂COGlu(O-Bz)N[(CH₂)₁₇CH₃]₂ (29) (RPR 123027 A)

Le produit (3) est couplé au produit (27) et déprotégé par la méthode G (Boc).Le produit final est purifié par CLHP semipréparative et les fractions analysées par CLHP analytique.
CLHP, Rt= 16.02 min,(H₂O/MeCN: 3 min [60/40], 3-20 min [0/100], 35 min [0/100]
MH⁺: 983
Spectre de R M N ¹H (400 MHz, (CD₃)₂SO d6, à une température de 413K, d en ppm) : 0,89 (t, J = 7 Hz, 6H : CH₃ des chaînes grasses) ; 1,30 (mt, 60H : (CH₂)₁₅ centraux des chaînes grasses) ; 1,55 (mf, 4H : 1 CH₂ de chaque chaîne grasse) ; 1.72 (mt, 4H : (CH₂)₂ centraux du butyle) ; de 1,75 à 2,00 (mt, 2H : CH₂ central du glutaryle) ; 1,99 (mt, 4H : CH₂ des propyles) ; 2,47 (t, J = 7 Hz, 2H : COCH₂ du glutaryle) ; 2,95 - 3,05 et 3,40 (3 mts, 16H en totalité : NCH₂ du butyle - NCH₂ des propyles et NCH₂ des chaînes grasses) ; 3,62 (s large, 2H : NCH₂CON) ; 4,85 (mt, 1H: CONCHCON du glutaryle) ; 5,14 (AB limite, J = 12 Hz, 2H : CH₂ du benzyle) ; 7,35 (mt, 5H : H Aromatiques du benzyle) ; 8,23 (mf, 1H : CONH du glutaryle).

### EXEMPLE 18

### Synthèse de NH₂(CH₂)₃NH(CH₂)₄NH(CH₂)₃NHCH₂COGlu(Galactosamide)N[CH₂)₁₇CH₃]₂ (31) (RPR 130596 A)

### a- BocNH(CH₂)₃NBoc(CH₂)₄NBoc(CH₂)₃NBocCH₂COGluN[(CH₂)₁₇CH₃]₂ (30)

Le produit (3) est couplé au produit (27) et le groupe de protection OBz de la chaîne latérale est clivé par la méthode H (Cl-Z) et utilisé sans autre purification.
CLHP, Rt= 22.84 min,(H₂O/MeCN: 3 min [60/40], 3-20 min [0/100], 35 min [0/100] MH⁺: 1293

### b-NH₂(CH₂)₃NH(CH₂)₄NH(CH₂)₃NHCH₂COGlu(Galactosamide)N[(CH₂)₁₇CH₃]₂ (31)

Le produit (30) est couplé avec du D(+)-Galactosamine chlorhydrate selon la méthode G. Le produit est purifié par CLHP semipréparative et les fractions analysées par CLHP analytique.
CLHP, Rt=13.71 min,(H₂O/MeCN: 3 min [60/40], 3-20 min [0/100], 35 min [0/100]
MH⁺: 1054

### EXEMPLE 19

### Synthèse de NH₂(CH₂)₃NH(CH₂)₄NH(CH₂)₃NHCH₂COGlu(Galactosamide)N[(CH₂)₁₇CH₃]₂ (32) (RPR 130595 A)

Le produit (30) est couplé avec du D(+)-Glucosamine chlorhydrate selon la méthode G. Le produit est purifié par CLHP semipréparative et les fractions analysées par CLHP analytique.
CLHP, Rt=12.27 min,(H₂O/MeCN: 3 min [60/40], 3-20 min [0/100], 35 min [0/100]
MH⁺: 1054

### EXEMPLE 20

### Synthèse de NH₂(CH₂)₃NH(CH₂)₄NH(CH₂)₃NHCH₂COGlu(Mannosamide)N[(CH₂)₁₇CH₃]₂ (33) (RPR 130598 A)

Le produit (30) est couplé avec du D(+)Mannosamine chlorhydrate selon la méthode G. Le produit est purifié par CLHP semipréparative et les fractions analysées par CLHP analytique.
CLHP, Rt=12.98 min,(H₂O/MeCN: 3 min [60/40], 3-20 min [0/100], 35 min [0/100]
MH⁺: 1054

### EXEMPLE 21

### Synthèse de NH₂(CH₂)₃NH(CH₂)₄NH(CH₂)₃NHCH₂COGlu(N(CH₃)₂)N[(CH₂)₁₇CH₃]₂ (34) (RPR 131111 A)

Le produit (30) est couplé avec de la Diméthylamine selon la méthode G. Le produit est purifié par CLHP semipréparative et les fractions analysées par CLHP analytique.
CLHP, Rt=14.44 min,(H20/MeCN: 3 min [60/40], 3-20 min [0/100], 35 min [0/100]
MH⁺: 920
Spectre de R M N ¹H (400 MHz, (CD₃)₂SO d6, d en ppm) : 0,89 (t, J = 7 Hz, 6H : CH₃ des chaînes grasses) ; 1,25 (mt, 60H : (CH₂)₁₅ centraux des chaînes grasses) ; 1,43 et 1,60 (2 mts, 2H chacun : 1 CH₂ de chaque chaîne grasse) ; 1,65 (mt, 4H : (CH₂)₂ centraux du butyle) ; 1,65 et de 1,85 à 2,00 (2 mts, 1H chacun : CH₂ central du glutaryle) ; 1,95 (mt, 4H : CH₂ des propyles) ; 2,32 (AB limite, 2H : COCH₂ du glutaryle) ; 2,80 et 2,92 (2s, 3H chacun : CON(CH₃)₂) ; de 2,85 à 3,05 (mt, 12H : NCH₂ du butyle - NCH₂ des propyles) ; 3,00 - 3,22 - 3,45 et 3,58 (4 mts, 1H chacun : NCH₂ des chaînes grasses) ; 3,78 (AB, J = 16 Hz, 2H : NCH₂CON) ; 4,75 (mt, 1H : CONCHCON du glutaryle) ; 8,72 (d, J = 7,5 Hz, 1H : CONH du glutaryle) ; 8,85 et de 8,90 à 9,15 (mfs échangeables).

### EXEMPLE 22

### Synthèse de NH₂(CH₂)₃NH(CH₂)₄NH(CH₂)₃NHCH₂COGlyN[(CH₂)₁₁CH₃]₂ (35) (RPR 122767 A )

Synthétisé de la même manière que le produit (6), mais en utilisant de la didodecylamine à la place de dioctadecylamine. Le produit est purifié par CLHP semipréparative et les fractions analysées par CLHP.
CLHP, Rt=9.54 min,(H₂O/MeCN: 3 min [60/40], 3-20 min [0/100], 35 min [0/100]
MH⁺: 653
Spectre de R M N ¹H (400 MHz, (CD₃)₂SO d6, à une température de 403K, d en ppm) : 0,93 (t, J = 7 Hz, 6H : CH₃ des chaînes grasses) ; 1,33 (mt, 36H : (CH₂)₉ centraux des chaînes grasses) ; 1,58 (mt, 4H : 1 CH₂ de chaque chaîne grasse) ; 1,75 (mt, 4H : (CH₂)₂ centraux du butyle) ; 1,95 et 2,00 (2 mts, 2H chacun : CH₂ central des propyles) ; 2,98 et 3,00 (2 mts, 12H en totalité : NCH₂ du butyle et NCH₂ des propyles) ; 3,30 (t, J = 7Hz, 4H : NCH₂ des chaînes grasses) ; 3,58 (s, 2H : NCH₂CON) ; 4,05 (s, 2H : CONCH₂CON du glycyle).

### EXEMPLE 23

### Synthèse de NH₂(CH₂)₃NH(CH₂)₄NH(CH₂)₃NHCH₂COGlyN[(CH₂)₁₂CH₃]₂ (36) (RPR 122774 A)

Synthétisé de la même manière que le produit (6), mais en utilisant de la ditridecylamine à la place de dioctadecylamine. Le produit est purifié par CLHP semipréparative et les fractions analysées par CLHP.
CLHP, Rt=10.64 min,(H₂O/MeCN: 3 min [60/40], 3-20 min [0/100], 35 min [0/100]
MH⁺: 681
Spectre de R M N ¹H (400 MHz, (CD₃)₂SO d6, à une température de 393K, d en ppm) : 0,91 (t, J = 7 Hz, 6H : CH₃ des chaînes grasses) ; 1,33 (mt, 40H : (CH₂)₁₀ centraux des chaînes grasses) ; 1,58 (mts, 4H : 1 CH₂ de chaque chaîne grasse) ; 1,75 (mt, 4H : (CH₂)₂ centraux du butyle) ; 2,00 (mt, 4H : CH₂ central des propyles) ; 2,98 et 3,08 (2 t, J = 7 Hz, 12H en totalité : NCH₂ du butyle et NCH₂ des propyles) ; 3,32 (t, J = 7 Hz, 4H : NCH₂ des chaînes grasses) ; 3,65 (s, 2H : NCH₂CON) ; 4,06 (d, J = 4 Hz, 2H : CONCH₂CON du glycyle) ; 8,60 (s large, 1H : CONH du glycyle).

### EXEMPLE 24

### Synthèse de NH₂(CH₂)₃NH(CH₂)₄NH(CH₂)₃NHCH₂COGlyN[(CH₂)₁₃CH₃]₂ (37) (RPR 122766 A )

Synthétisé de la même manière que le produit (6), mais en utilisant de la ditetradecylamine à la place de dioctadecylamine. Le produit est purifié par CLHP semipréparative et les fractions analysées par CLHP. Le produit est purifié par CLHP semipréparative et les fractions analysées par CLHP.
CLHP, Rt=9.92 min,(H₂O/MeCN: 3 min [60/40], 3-20 min [0/100], 35 min [0/100]
MH⁺: 709
Spectre de R M N ¹H (400 MHz, (CD₃)₂SO d6, à une température de 393K, d en ppm) : 0,90 (t, J = 7 Hz, 6H : CH₃ des chaînes grasses) ; 1,31 (mt, 44H : (CH₂)₁₁ centraux des chaînes grasses) ; 1,58 (mt, 4H : 1 CH₂ de chaque chaîne grasse) ; 1,76 (mt, 4H : (CH₂)₂ centraux du butyle) ; 2,00 (mt, 4H : CH₂ central des propyles) ; 2,98 et 3,08 (respectivement mt et t, J = 7 Hz, 12H en totalité : NCH₂ du butyle et NCH₂ des propyles) ; 3,30 (t, J = 7 Hz, 4H : NCH₂ des chaînes grasses) ; 3,65 (s, 2H : NCH₂CON) ; 4,06 (d, J = 4 Hz, 2H : CONCH₂CON du glycyle) ; 8,10 (mf, 1H : CONH du glycyle).

### EXEMPLE 25

### Synthèse NH₂(CH₂)₃NH(CH₂)₄N[(CH₂)₃NH₂]CH₂COGlyN[(CH₂)₁₇CH₃]₂ (39) (RPR 126096 A)

### a- Synthèse de BocNH(CH₂)₃NBoc(CH₂)₄N[(CH₂)₃NHBoc]CH₂CO₂H (38)

Pendant la synthèse du produit (3), on récupère lors de la purification sur SiO₂ le sous produit (38). Le rendement est de 8 %.
CCM Rf= 0.32 (CHCl₃/MeOH, 9:1)
MH⁺: 561
Spectre de R M N ¹H (400 MHz, (CD₃)₂SO d6, d en ppm) : de 1.30 àl.60 (mt, 4H : (CH₂)₂ centraux du butyle) ; 1,40 (s, 27H : C(CH₃)₃) ; 1,56 (mt, 4H : CH₂ des propyles) ; 2,68 et 3,11 (respectivement t large et t, J = 7 Hz, 4H chacun : NCH₂ du butyle et NCH₂ des propyles) ; 2,90 et 2,96 (2 q, J = 7 Hz, 2H chacun : BocNHCH₂ des propyles) ; 3,18 (s, 2H : NCH₂COO).

### b-NH₂(CH₂)₃NH(CH₂)₄N[(CH₂)₃NH₂]CH₂COGlyN[(CH₂)₁₇CH₃]₂ (39)

Les produits (38) et (5) sont couplés selon la méthode G. Le produit est purifié par CLHP semipréparative et les fractions analysées par CLHP.
CLHP, Rt=13.60 min,(H₂O/MeCN: 3 min [60/40], 3-20 min [0/100], 35 min [0/100]
MH⁺: 821
Spectre de R M N ¹H (400 MHz, (CD₃)₂SO d6 avec ajout de quelques gouttes de CD₃COOD d4, d en ppm) : 0,87 (t, J = 7 Hz, 6H : CH₃ des chaînes grasses) ; 1,28 (mt, 60H : (CH₂)₁₅ centraux des chaînes grasses) ; 1,46 et 1,54 (2 mts, 2H chacun : 1 CH₂ de chaque chaîne grasse) ; 1.63 (mt, 4H : (CH₂)₂ centraux du butyle) ; 1,91 (mt, 4H : CH₂ des propyles) ; de 2,85 à 3,15 (mt, 12H : NCH₂ du butyle et NCH₂ des propyles) ; 3,24 (mt, 4H : NCH₂ des chaînes grasses) ; 3,76 (mf, 2H : NCH₂CON) ; 4,05 (s large, 2H : CONCH₂CON du glycyle).

### EXEMPLE 26

### Synthèse de NH₂(CH₂)₃NH(CH₂)₄NH(CH₂)₃NH(CH₂)₃CON[(CH₂)₁₇CH₃]₂ (41) (RPR 122786 A)

### a-BocNH(CH₂)₃NBoc(CH₂)₄NBoc(CH₂)₃NBoc(CH₂)₃CO₂H (40)

On synthétise le produit (40) en faisant une réduction alkylative sur la spermine en présence de NaCNBH₃ et de succinic semialdehyde en solution.

Dans un ballon de 200 ml, on chargel.8 gr de spermine, 60 ml de méthanol et 0.138 gr de NaCNBH₃ La solution est soumise à une vive agitation magnétique . Par l'intermédiaire d'une ampoule isobare on coule en 100 minutes une solution de 5.5 ml de succinic semialdehyde (15 %) avec 30 ml de méthanol. On maintient l'agitation 100 minutes. On protège les amines par le groupe Boc de la manière suivante: on coule sur le milieu 2.8 ml de TEA puis 8.8 gr de Ditertiobutyl dicarbonate solubilisé dans 30 ml de Méthanol. On maintient la nuit sous agitation. Le milieu est concentré in vacuo, le produit est repris dans l'acétate d'éthyle et extrait avec trois fractions de 50 ml NaHCO₃, les phases aqueuses sont réunies et rincées avec éther (3x100 ml). Le pH de la phase aqueuse est descendu 3 avec KHSO4, on apprécie une turbidité due à la précipitation du produit 41, le mélange est extrait avec acétate d'éthyle (3x100ml). La phase organique est séchée sur MgSO₄ et évaporée in vacuo. Le produit est utilisé sans autre purification.

### b- NH₂(CH₂)₃NH(CH₂)₄NH(CH₂)₃NH(CH₂)₃CON[(CH₂)₁₇CH₃]₂ (41)

Le produit (40) et la dioctadécylamine sont couplés selon la méthode G. Le produit est purifié par CLHP semipréparative et les fractions analysées par CLHP.
CLHP, Rt=15.04 min,(H₂O/MeCN: 3 min [60/40], 3-20 min [0/100], 35 min [0/100]
MH⁺: 792
Spectre de R M N ¹H (400 MHz, (CD₃)₂SO d6, à une température de 383K, d en ppm) : 0,85 (t, J = 7 Hz, 6H : CH₃ des chaînes grasses) ; 1,22 (mt, 60H : (CH₂)₁₅ centraux des chaînes grasses) ; 1,48 (mf, 4H : 1 CH₂ de chaque chaîne grasse) ; 1.72 (mt, 4H : (CH₂)₂ centraux du butyle) ; 1,88 (mt, 2H : CH₂ central de l'aminopentanoyle) ; 1,99 (mt, 4H : CH₂ des propyles) ; 2,42 (t, J = 7 Hz, 2H : COCH₂ de l'aminopentanoyle) ; 2,96 - 3,03 et 3,22 (3 mts, 18H en totalité : NCH₂ de l'aminopentanoyle - NCH₂ du butyle - NCH₂ des propyles et NCH₂ des chaînes grasses).

### EXEMPLE 27

### Synthèse de NH₂(CH₂)₃NH(CH₂)₄NH(CH₂)₃NHCH₂CONH(CH₂)₅CON[(CH₂)₁₇CH₃]₂ (42) (RPR 128506 A)

Synthétisé de la même manière que le produit (6), mais en utilisant Boc 6-aminocaproïc acide à la place de BocGly. Le produit est purifié par CLHP semipréparative et les fractions analysées par CLHP.
CLHP, Rt=13.94 min,(H₂O/MeCN: 3 min [60/40], 3-20 min [0/100], 35 min [0/100]
MH⁺: 877
Spectre de R M N ¹H (300 MHz, (CD₃)₂SO d6, d en ppm) : 0,87 (t, J = 7 Hz, 6H : CH₃ des chaînes grasses) ; 1,28 (mt, 60H : (CH₂)₁₅ centraux des chaînes grasses) ; 1,48 (mt, 10H : 1 CH₂ de chaque chaîne grasse et (CH₂)₃ centraux de l'aminohexanoyle) ; 1,65 (mt, 4H : (CH₂)₂ centraux du butyle) ; 1,95 (mt, 4H : CH₂ des propyles) ; 2,27 (t, J = 7 Hz, 2H : COCH₂ de l'aminohexanoyle) ; de 2,85 à 3,30 (mts, 18H : NCH₂ de l'aminohexanoyle - NCH₂ du butyle - NCH₂ des propyles et NCH₂ des chaînes grasses) ; 3,70 (s large, 2H : NCH₂CON) ; de 7,90 à 9,10 (mfs échangeables).

### EXEMPLE 28

### Synthèse de NH₂(CH₂)₃NH(CH₂)₄NH(CH₂)₃NHCH₂COGlu(11-amide-undécanyl,hepta,O acétyl lactose) N[(CH₂)₁₇CH₃]₂ (43) (RPR 130765 A)

Le produit (30) est couplé avec de le 11 aminoundécanyl hepta O acétyl lactose selon la méthode G. Le produit est purifié par CLHP semipréparative et les fractions analysées par CLHP analytique.
CLHP, Rt=15.91 min,(H₂O/MeCN: 3 min [60/40], 3-20 min [0/100], 35 min [0/100]
MH⁺: 1680

### EXEMPLE 29

### Synthèse de NH₂(CH₂)₃NH(CH₂)₄NH(CH₂)₃NHCH₂COAsm(β-NAc(Ac)₃)N[(CH₂)₁₇CH₃]₂ (RPR 131283 A) (45)

### a- Fmoc-Asm-β-Glc-NAc(Ac)₃-dioctadécylamine (44)

Le produit est synthétisé par couplage de Fmoc-Asm-β-Glc-NAc(Ac)₃-OH et de dioctadécylamine par la méthode F
CCM Rf= 0.67 (CHCl₃/MeOH, 9:1)
CLHP, Rt=25.31 min,(H₂O/MeCN: 3 min [60/40], 3-20 min [0/100], 35 min [0/100]

### b-NH₂(CH₂)₃NH(CH₂)₄NH(CH₂)₃NHCH₂CO Asm (β-NAc(Ac)₃)N[(CH₂)₁₇CH₃]₂ (45)

### Clivage du groupement Fmoc du produit (45).

On coule sur 0.7 gr de produit (45) 20 ml de DMF et 2 ml de Diéthylamine. Aprés 6 heures de maintient sous agitation le milieu est concentré in vacuo

Le produit obtenu est couplé au produit (3) selon la méthode G. Le produit est purifié par CLHP semipréparative et les fractions analysées par CLHP analytique.
CLHP, Rt=15.35 min,(H₂O/MeCN: 3 min [60/40], 3-20 min [0/100], 35 min [0/100]
MH⁺: 1207

### EXEMPLE 30

### Synthèse en solution du produit (6) à grande échelle.

On synthétise le produit (6) en faisant une réduction alkylative sur la spermine en présence de NaCNBH₃ et de l'acide glioxylique en solution.

Dans un ballon de 2 l, on charge 18.2 gr de spermine, 500 ml de méthanol et 2 gr de NaCNBH₃ . La solution est soumise à une vive agitation magnétique. Par l'intermédiaire d'une ampoule isobare on coule en 100 minutes une solution de 8.45 gr. d'acide glioxylic dans 300 ml de méthanol. On maintient l'agitation pendant une nuit. On protège les amines par le groupe Boc de la manière suivante: on coule sur le milieu 14 ml de TEA puis 100 gr de Ditertiobutyl dicarbonate solubilisé dans 200 ml THF. On maintient la nuit sous agitation. Le milieu est concentré in vacuo, le produit est repris dans acétate d'éthyle (250 ml), rincé avec KHSO₄ (6x100 ml) puis avec une solution saturée de NaCl (3x100), séché sur MgSO₄ et évaporée in vacuo. Le produit est purifié sur colonne de silice avec pour eluant CHCl₃/MeOH (9:1). Les fractions contenant le produit sont identifiées par CCM, regroupées et évaporées in vacuo pour obtenir 10 gr. du produit (6) (rendement de 17 % pour la synthèse total).

Les analyses de CLHP analytique, spectre de masse et RMN sont identiques à celles du produit obtenue par la méthode de phase solide.

### EXEMPLE 31

### Influence du rapport de charge (amines/phosphates) sur l'efficacité de transfection (7) RPR 120534A. (6) RPR 120535A et (9) RPR 120531A

Des échantillons de 1.10⁵ cellules [NIH 3T3, 3LL ou SMClapin] en phase exponentielle de croissance sur 2 cm² sont traités par des solutions lipofectant/pCMV-LUC, présentant des rapports de charges variables, pendant 2 heures à 37°C sous 5% CO₂ ; chaque échantillon reçoit 2 µg d'acide nucléique. La recherche de l'expression du gène reporter est effectuée après addition de sérum de veau foetal à 8% final suivie d'une incubation de 40 heures dans l'étuve à CO₂.

L'activité luciférase est dosée par l'émission de lumière [RLU = relative light unit] en présence de luciférine, coenzyme A et ATP pendant 10 secondes et rapportée à 2000 cellules traitées. Les résultats obtenus sont reportés dans les figures (1), (2) et (3).

De l'observation de ces figures, il ressort clairement que la présence d'une glycine dans le bras «spacer» entre la partie lipidique et la polyamine permet d'obtenir une meilleure efficacité de transfection pour des ratio nanomoles lipide cationique/µg ADN faibles.

### EXEMPLE 32

### Influence du rapport de charge (amines/phosphates) sur l'efficacité de transfection (20) RPR 120527A, (19) RPR 120528A, (17) RPR 120526A et (16) RPR 120525A.

Des échantillons de 1.10⁵ cellules NIH 3T3 en phase exponentielle de croissance sur 2 cm² sont traités par des solutions lipofectant/pCMV-LUC, présentant des rapports de charges variables, pendant 2 heures à 37°C sous *5%* CO₂ ; chaque échantillon reçoit 1 µg d'acide nucléique. La recherche de l'expression du gène reporter est effectuée après addition de sérum de veau foetal à 8% final suivie d'une incubation de 40 heures dans l'étuve à CO₂.

L'activité luciférase est dosée dans le surnageant obtenu après lyse des cellules par l'émission de lumière [RLU = relative light unit] pendant 10 secondes et rapportée au mg de protéine. Les résultats obtenus sont reportés dans la figure (4). L'avantage de la présence du résidu glycine dans le bras « spacer » est encore mis en évidence dans cet exemple.

### EXEMPLE 33

### Influence de la longueur du spacer sur l'efficacité de transfection (6) RPR 120535, (41) RPR 122786 et (42) RPR 128506.

Des échantillons de 1.10⁵ cellules [NIH3T3 et HeLa] en phase exponentielle de croissance sur 2 cm² sont traitées par des mélanges lipide cationique/pCMV-Luc, présentant des concentrations variables en lipide cationique, à 37°C en atmosphère humide sous 5% CO₂, pendant 2 heures en absence de protéines sériques. Le milieu de croissance des cellules est ensuite supplémenté par du sérum de veau foetal à 8% final et la mesure de l'expression du transgène est effectuée après 40 heures supplémentaires d'incubation dans l'étuve à CO₂.

Les caractéristiques structurales des spacers sont comme suit:

| | |
|---|---|
| N° RPR | Spacer |
| 122786 | - |
| 120535 | Gly |
| 128506 | NH₂(CH₂)₅CO |

Le tableau I suivant reportent les résultats obtenus qui sont exprimés pour les efficacités maximum obtenues avec chacun des produits étudiés.

**TABLEAU I**

| | lipide cationique | cellules HeLa | cellules NIH3T3 |
|---|---|---|---|
| expérience 1 | RPR120535 (6) | 1,2.10⁶ ± 9,7.10⁴ (4) | 8,7.10⁷ ± 4,5.10⁶ (4) |
| | RPR122786 (41) | 2,3.10⁶ ± 1,6.10⁵ (8) | 4,6.10⁷ ± 5,0.10⁶ (8) |
| | | | |
| expérience 2 | RPR120535 (6) | 2,4.10⁶ ± 3,2.10⁵ (6) | 7,2.10⁷ ± 8,3.10⁶ (6) |
| | RPR128506 (42) | 1,8.10⁶ ± 1,3,10⁵ (6) | 5,0.10⁷ ± 1,5.10⁶ (6) |

Les efficacités de transfecton sont données en RLU/10s./2.103 cellules traitées. Entre parenthèses sont indiqués les ratio nanomoles lipid/µg ADN.

Des plasmides différents ont été utilisés pour les expériences 1 et 2, à dose de 1µg et 0,5 µg ADN/1.105 cellules respectivement dans les expériences 1 et 2.

### Exemple 34 :

### Influence de la structure du spacer sur l'efficacité de transfection (6) RPR 120535.

Dans des conditions d'expérimentation identiques à celles décrites dans l'exemple précédent, mais avec l'introduction d'une lignée supplémentaire (cellules 3LL), nous avons comparé les efficacités de transfection obtenues avecle lipide cationique (6) RPR120535 modifié par substitution sur le « spacer » avec un groupement type Arg, type Lys ou type Glu.

Les structures des différents spacers sont comme suit:

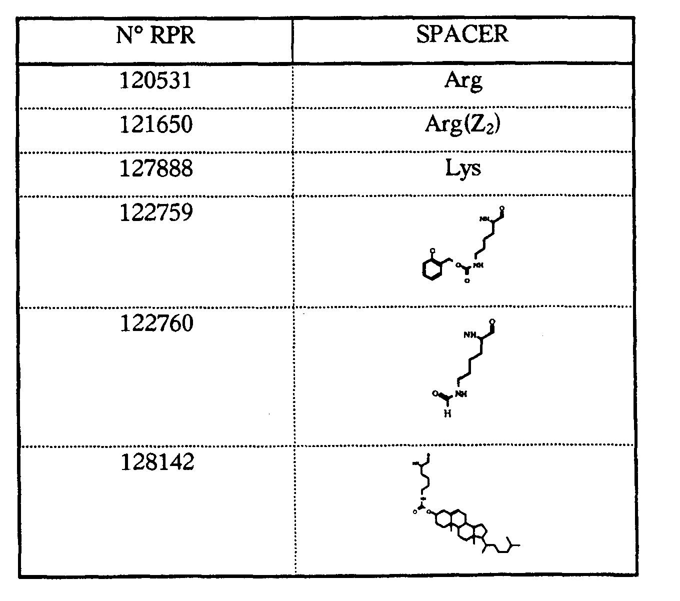

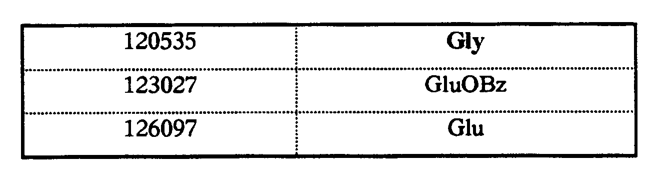

Les efficacités de transfection sont données en RLU/10s./2.103 cellules traitées.

Des plasmides différents ont été utilisés pour les expériences 1 et 2, à dose de 0,5µg et 1µg ADN/1.105 cellules respectivement dans les expériences 1 et 2. De l'analyse des résultats, il ressort que selon les cellules considérées, la présence d'une chaîne d'acide aminé de préférence substituée induit une meilleur efficacité de transfection.

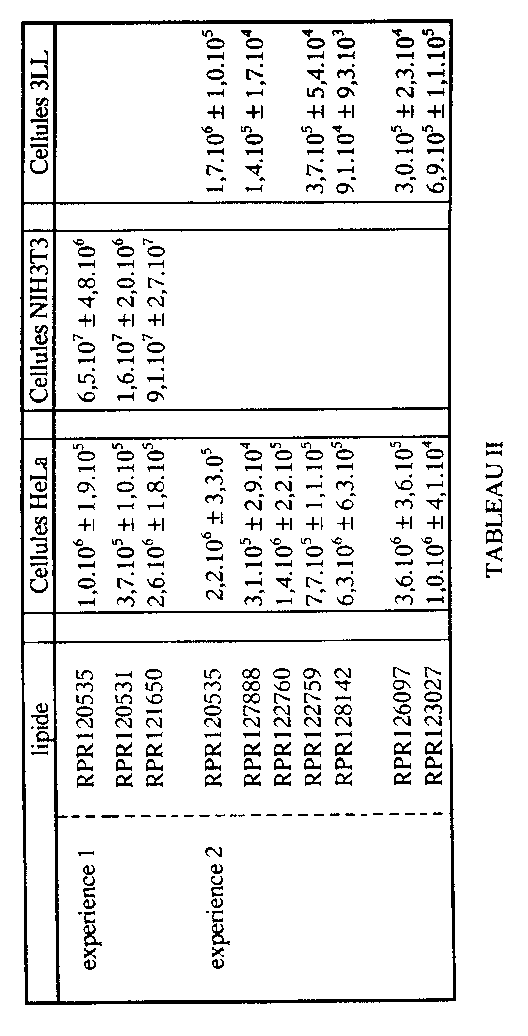

### EXEMPLE 34

### Influence de la présence de DOPE dans le mélange lipofectant/ADN (9) RPR 120531A

Selon le mème protocole que celui utilisé dans l'exemple 31 il est ajouté de la DOPE (Dioleoylphosphatidylethanolamine) au lipide cationique **(9)** *RPR 120531A* à des rapports molaires variables avant addition du DNA dans le mélange de transfection.

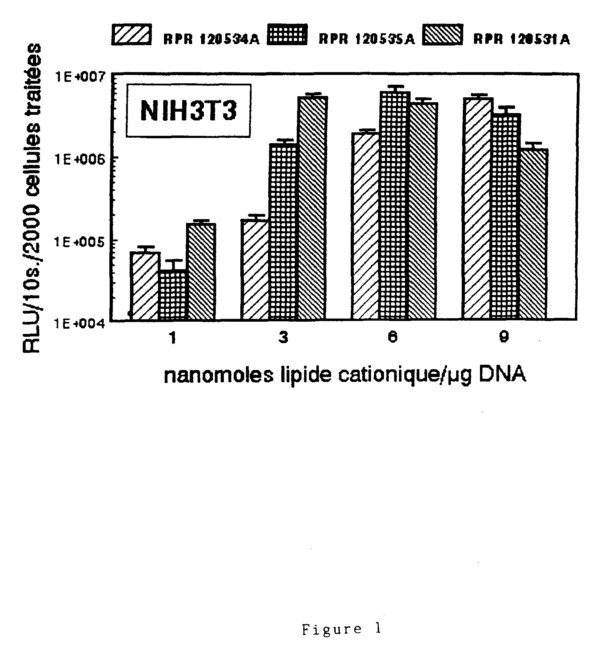

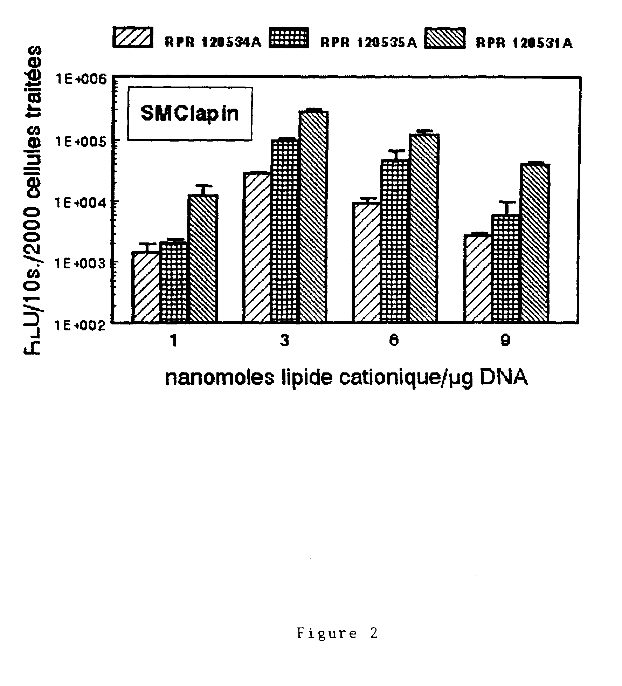

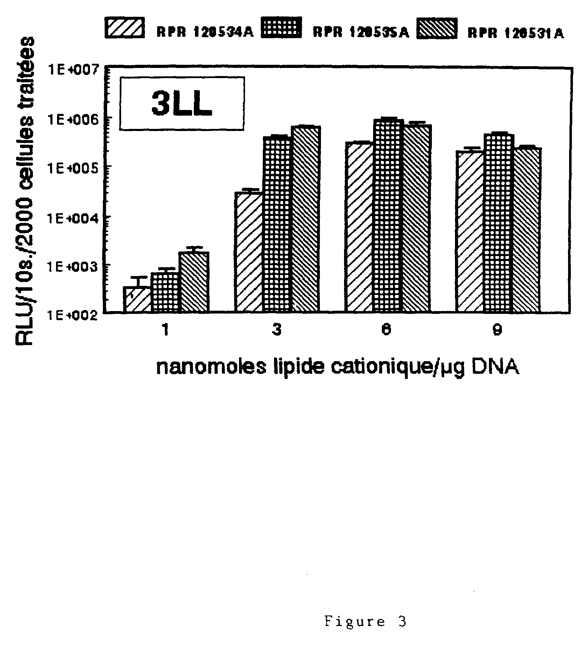

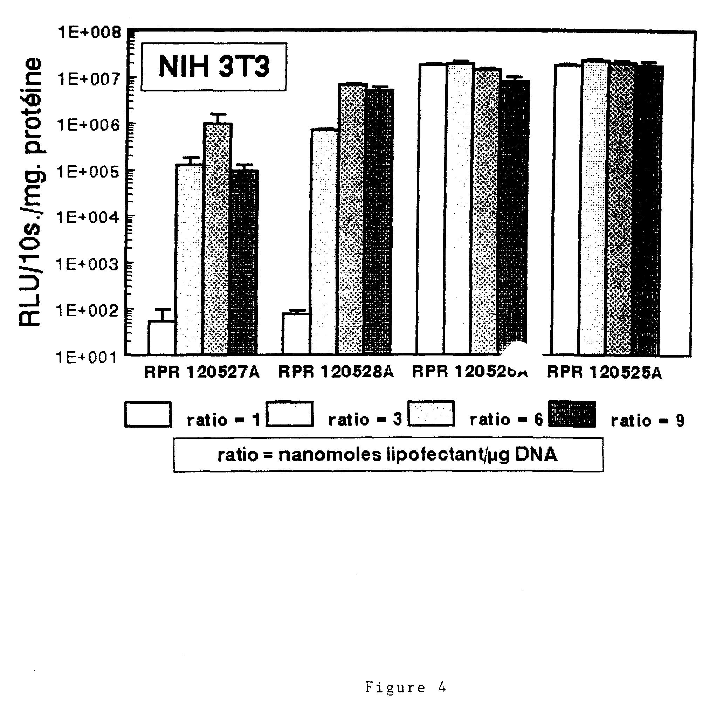

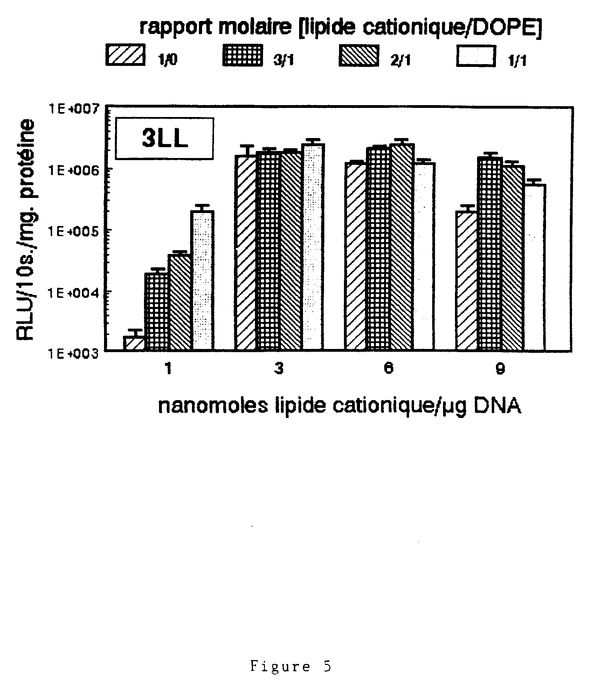

L'activité luciférase est dosée dans le surnageant après lyse des cellules et rapportée au mg de protéine (figure 5).

La présence de DOPE dans les mélanges lipofectants permet d'améliorer l'efficacité de transfection quand la concentration en **(9)** *RPR 120531A* est faible.

### EXEMPLE 35

### Effet du sérum sur l'efficacité de transfection (6) RPR 120535A, (9) RPR 120531A et (10) RPR 121650A

Des échantillons de 1.10⁵ cellules [NIH 3T3 ou Hela] en phase exponentielle de croissance sur 2 cm² sont traités par des solutions lipofectant/pCMV-LUC (3 nanomoles lipofectant/µg ADN) en absence de sérum pendant 2 heures ou en présence de sérum dans le milieu de culture. Dans cet exemple chaque échantillon reçoit 2 µg d'ADN. L'expression de la luciférase est recherchée dans le surnageant des lysats cellulaires, exprimée en RLU/10s. et rapportée au mg de protéine. De l'analyse des résultats, il ressort que la présence de sérum n'a pas d'effet notable sur la transfection.

### EXEMPLE 36

### Influence de la concentration en acide nucléique dans les mélanges ADN/lipofectant (20)RPR 120527A, (19)RPR 120528A, (17) RPR 120526A et (16) RPR 120525A

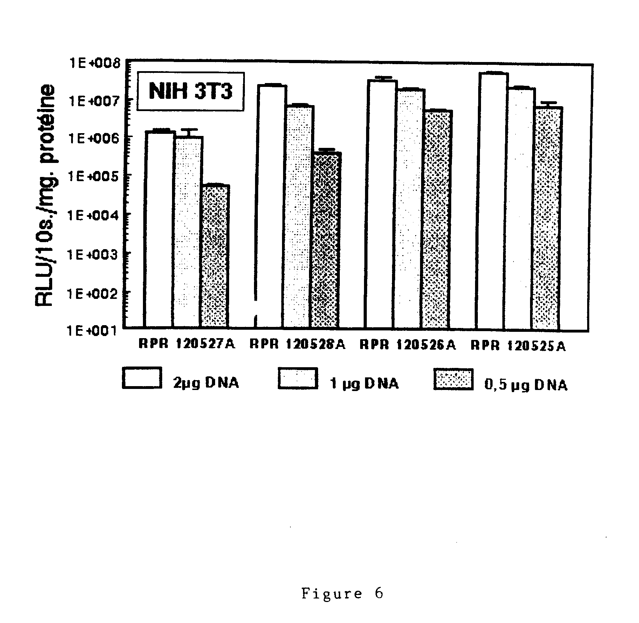

Dans les conditions décrites dans l'exemple 31 des échantillons de cellules NIH 3T3 sont transfectées dans des conditions où les ratios nanomoles lipofectant/µg d'ADN sont optimisés - voir exemple 32 - [ratio = 6 pour **(20)***RPR 120527A* et **(19)***RPR 120528A -* ratio = 3 pour **(17)***RPR 120526A* et **(16)***RPR 120525A*]*.* Les quantités d'ADN apportées à chaque échantillon varient de 0,5 à 2 µg. Les résultats sont reportés dans la figure 6.

La transfection de 1.10⁵ cellules en phase exponentielle de croissance avec 1 µg d'ADN plasmidique semble un bon choix ; en effet l'augmentation de la quantité d'ADN utilisé entraîne une augmentation de la concentration en lipide cationique au contact des cellules et donc des problèmes de toxicité dans certains cas. A plus faible concentration en ADN la proportionalité avec l'efficacité de transfection n'est plus obtenue.

### EXEMPLE 37

### Essais de transfection in vivo avec des lipopolyamines selon l'invention.

On procède à une injection d'une solution contenant une lipopolyamine selon l'invention, préparée comme décrit ci-dessus, dans la tumeur 7 jours après implantation, la souris étant anesthésiée avec un mélange Kétamine + Xylazine. Deux jours après l'injection, on prélève du tissus tumoral, qui est pesé puis haché et broyé dans 500 µl tampon de lyse (Promega Cell Lysis Buffer E153 A). Après centrifugation (20 000 g pendant 10 minutes), on prélève 10 µl qui servent à l'évaluation de l'activité luciférase par mesure de l'émission lumineuse totale obtenue après mélange avec 50 µl de réactif (Promega Luciferase Assay Substrate ) dans un luminomêtre Lumat LB 9501 (Berthold), intégration sur 10 secondes.L'activité résultante est exprimée en RLU (Relative Lights Units) estimés dans la totalité du surnageant de lyse tumorale, ou en RLU par µg d'ADN injecté, le tableau III rend compte des résultats obtenus.

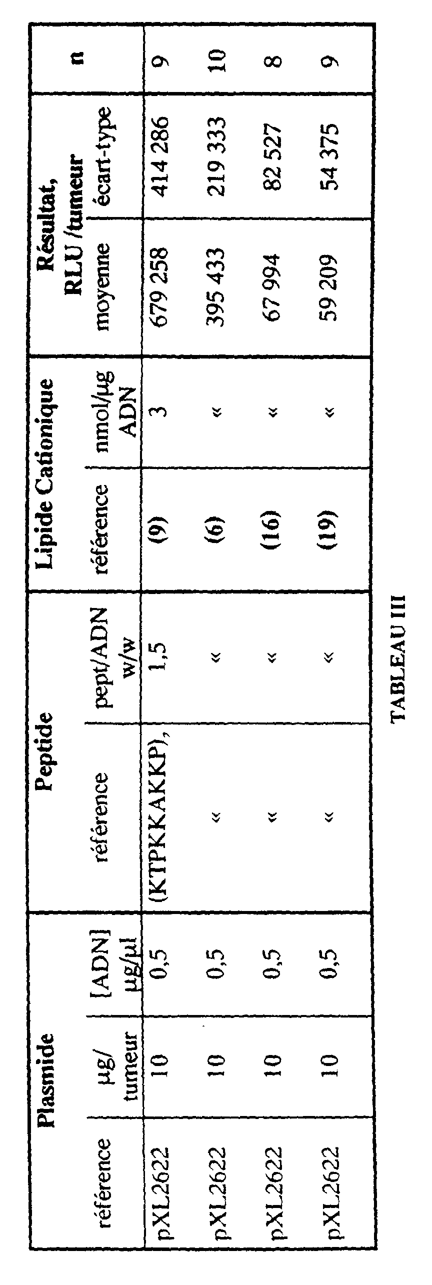

## Revendications

1. Lipopolyamine, sous forme D, L ou LD et ses sels, caractérisée en ce qu'elle est représentée par la formule générale I 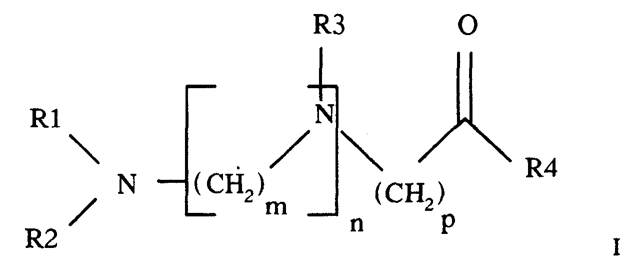 Dans laquelle :
R1, R2 et R3 représentent indépendamment l'un de l'autre un atome d'hydrogène ou un groupement -(CH2)q-NRR' avec
q pouvant varier entre 1, 2, 3, 4, 5 et 6 ceci de manière indépendante entre les différents groupements R1, R2 et R3 et
R et R' représentant indépendamment l'un de l'autre un atome d'hydrogène ou un groupement -(CH₂)q'-NH₂, q' pouvant varier entre 1, 2, 3, 4, 5 et 6 ceci de manière indépendante entre les différents groupements R et R',
m, n et p représentent, indépendamment l'un de l'autre, un nombre entier pouvant varier entre 0 et 6 avec lorsque n est supérieur à 1, m pouvant prendre des valeurs différentes et R3 des significations différentes au sein de la formule générale I et
R4 représente un groupement de formule générale II 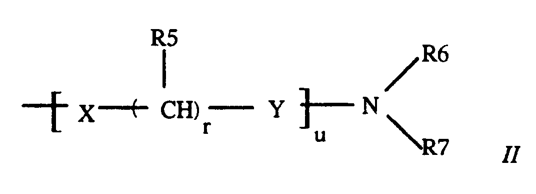 dans laquelle:
R6 et R7 représentent indépendamment l'un de l'autre un atome d'hydrogène ou un radical aliphatique, saturé ou non, en C10 à C22 avec au moins l'un des deux groupements étant différent de l'hydrogène,
u est un nombre entier choisi entre 0 et 10 avec lorsque u est un entier supérieur à 1 R5, X, Y et r pouvant avoir des significations différentes au sein des différents motifs [ X-(CHR5)r-Y]
X représente un atome d'oxygène, de soufre ou un groupement amine monoalkylé ou non,
Y représente un groupement carbonyle ou un groupement méthylène
R5 représente un atome d'hydrogène ou une chaîne latérale d'acide aminé naturel , le cas échéant substituée et
r représente un entier variant entre 1 et 10 avec lorsque r est égal à 1, R5 représentant une chaîne latérale d'acide aminé naturel substitué ou non et lorsque r est supérieur à 1, R5 représentant un atome d'hydrogène.

2. Lipopolyamine selon la revendication 1 caractérisée en ce qu'elle est représentée par l'une des sous formules suivantes 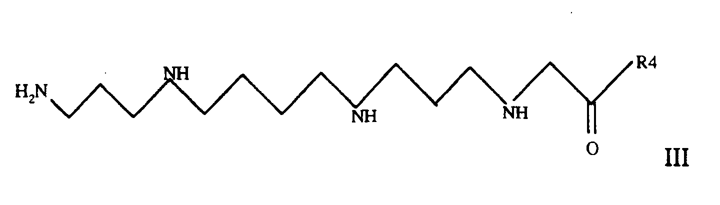 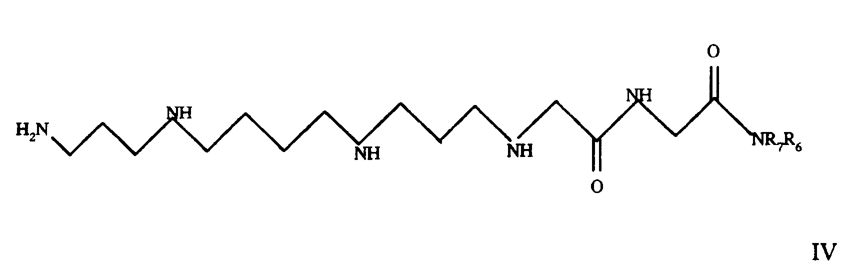 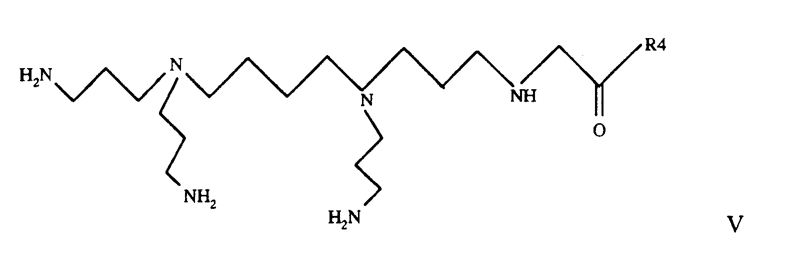 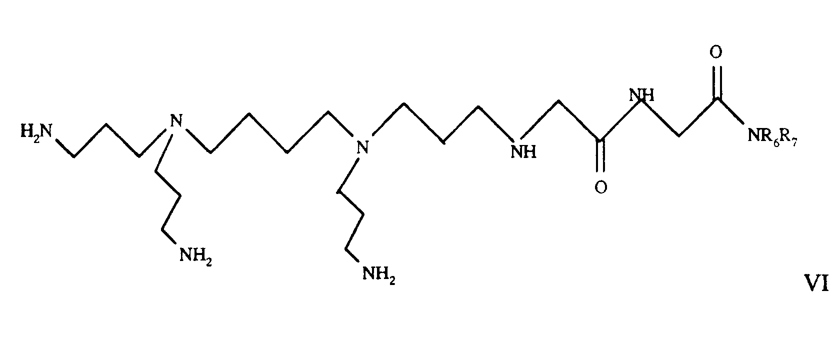 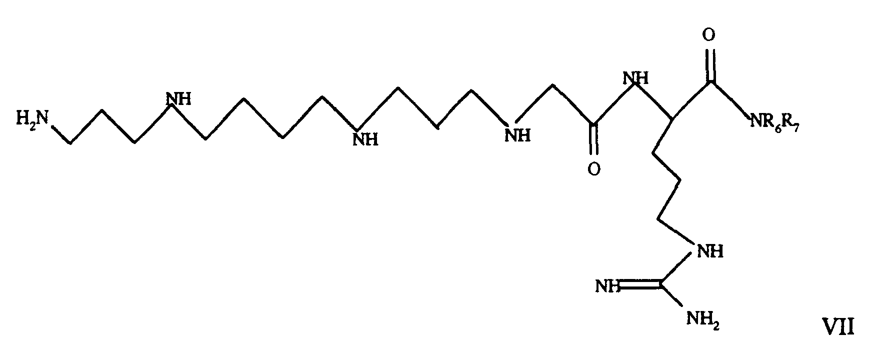 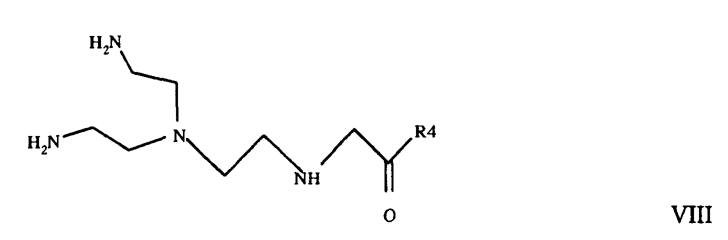 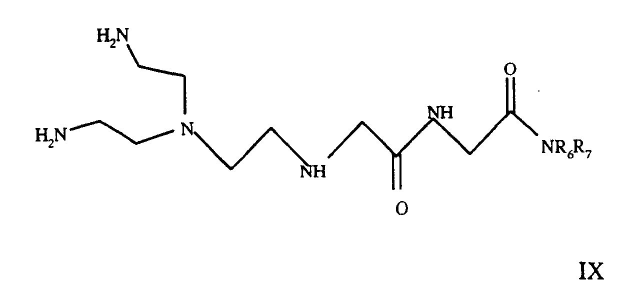 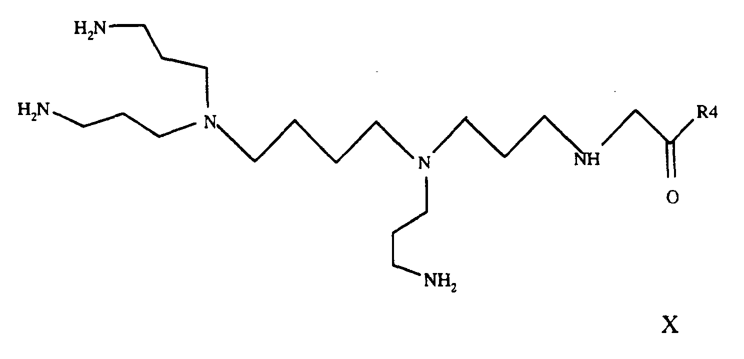 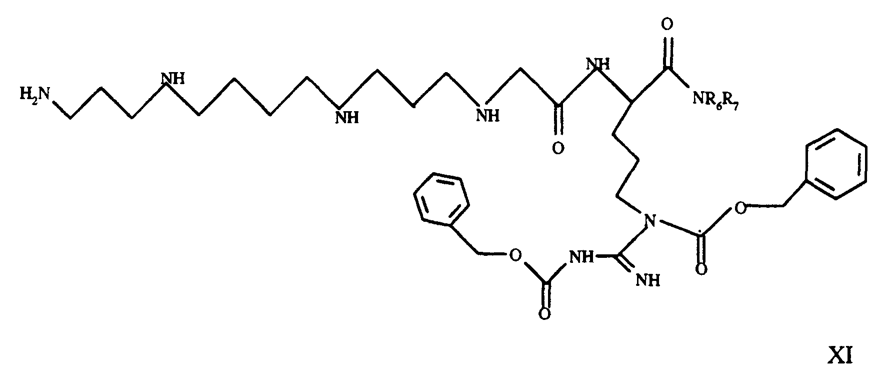 dans lesquelles R4, R6 et R7 sont définis comme en revendication 1.

3. Lipopolyamine selon la revendication 2 caractérisée en ce que R4 y représente un groupement NR6R7 avec R6 et R7 figurant dans les sous formules III à XII un groupement identique choisi parmi (CH₂)₁₇ CH₃, (CH₂)₁₁ CH₃, (CH₂)₁₃ CH₃ ou (CH₂)₁₂ CH₃.

4. Lipopolyamine selon l'une des revendications précédentes caractérisée en ce qu'elle est associée à un élément de ciblage extra ou intracellulaire.

5. Lipopolyamine selon la revendication 4 caractérisée en ce qu'elle incorpore l'élément de ciblage au niveau de la chaîne latérale d'acide aminé figurée par le substituant R5.

6. Lipopolyamine selon la revendication 4 ou 5 caractérisée en ce qu'il s'agit d'un ligand de récepteur cellulaire présent à la surface du type cellulaire ciblé.

7. Lipopolyamine selon l'une des revendications 4 à 6 caractérisée en ce que cet élément de ciblage est choisi parmi des sucres, des peptides tels que des anticorps ou fragments d'anticorps, des ligands de récepteurs cellulaires ou des fragments de ceux-ci, des récepteurs ou fragments de récepteurs tels que des ligands de récepteurs de facteurs de croissance, de récepteurs de cytokines, de récepteurs de lectines cellulaires ou de récepteurs de protéines d'adhésion de type intégrines, de récepteur de la transferrine, des lipides HDL ou LDL et/ou des oligonucléotides.

8. Lipopolyamine selon l'une des revendications 4 ou 5 caractérisée en ce que cet élément de ciblage est représentée par une séquence signal de localisation nucléaire.

9. Lipopolyamine selon l'une des revendications précédentes caractérisée en ce qu'elle incorpore un agent marqueur de type biotine, rhodamine, folate ou une séquence peptidique ou pseudopeptidique linéaire ou cyclique comportant l'épitope Arg-Gly-Asp au niveau de la chaine latérale d'acide aminé figurée par R5.

10. Lipopolyamine selon l'une des revendications précédentes caractérisée en ce qu'elle est choisie parmi :
H₂N(CH₂)₃NH(CH₂)₄NH(CH₂)₃NHCH₂COGlyN[(CH₂)₁₇-CH₃]₂
H₂N(CH₂)₃NH(CH₂)₄NH(CH₂)₃NHCH₂CON[(CH₂)₁₇-CH₃]₂
H₂N(CH₂)₃NH(CH₂)₄NH(CH₂)₃NHCH₂COArgN[(CH₂)₁₇-CH₃]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(rhodamine)N[(CH₂)₁₇-CH₃]₂
H₂N(CH₂)₃NH(CH₂)₄NH(CH₂)₃NHCH₂COLys(biotinyl)N[(CH₂)₁₇-CH₃]₂
{H₂N(CH₂)₃}₂N(CH₂)₄N{(CH₂)₃NH₂}(CH₂)₃NHCH₂COGlyN[(CH₂)₁₇-CH₃]₂
{H₂N(CH₂)₃}₂N(CH₂)₄N{(CH₂)₃NH₂}(CH₂)₃NHCH₂CON[(CH₂)₁₇-CH₃]₂
{H₂N(CH₂)₂}₂N(CH₂)₂NHCH₂COGlyN[(CH₂)₁₇-CH₃]₂
{H₂N(CH₂)₂}₂N(CH₂)₂NHCH₂CON[(CH₂)₁₇-CH₃]₂
H₂N(CH₂)₃NH(CH₂)₄NH(CH₂)₃NHCH₂COGlyN[(CH₂)₁₇]₂
H₂N(CH₂)₃NH(CH₂)₄NH(CH₂)₃NHCH₂CON[(CH₂)₁₇]₂
H₂N(CH₂)₃NH(CH₂)₄NH(CH₂)₃NHCH₂COArgN[(CH₂)₁₇]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(rhodamine)N[(CH₂)₁₇-CH₃]₂
H₂N(CH₂)₃NH(CH₂)₄NH(CH₂)₃NHCH₂COLys(biotinyl)N[(CH₂)₁₇-CH₃]₂
{H₂N(CN₂)₃}₂N(CH₂)₄N{(CH₂)₃NH₂}(CH₂)₃NHCH₂COGlyN[(CH₂)₁₇-CH₃]₂
{H₂N(CH₂)₃}₂N(CH₂)₄N{(CH₂)₃NH₂}(CH₂)₃NHCH₂CON[(CH₂)₁₇-CH₃]₂
{H₂N(CH₂)₂}₂N(CH₂)₂NHCH₂COGlyN[(CH₂)₁₇-CH₃]₂
{H₂N(CH₂)₂}₂N(CH₂)₂NHCH₂CON[(CH₂)₁₇-CH₃]₂
NH₂(CH₂)₃NH(CH₂)₄N[(CH₂)₃NH₂]CH₂COGlyN[(CH₂)₁₇CH₃]₂
NH₂(CH₂)₃NH(CH₂)₄NH(CH₂)₃NHCH₂COLysN[(CH₂)₁₇CH₃]₂
NH₂(CH₂)₃NH(CH₂)₄NH(CH₂)₃NHCH₂COLys [Cl-Z]N[(CH₂)₁₇CH₃]₂
NH₂(CH₂)₃NH(CH₂)₄NH(CH₂)₃NHCH₂COLys [CHO]N[(CH₂)₁₇CH₃]₂
NH₂(CH₂)₃NH(CH₂)₄NH(CH₂)₃NHCH₂COLys [Cholesteryl]N[(CH₂)₁₇CH₃]₂
NH₂(CH₂)₃NH(CH₂)₄NH(CH₂)₃NHCH₂COLys [Arachidonyl]N[(CH₂)₁₇CH₃]₂
NH₂(CH₂)₃NH(CH₂)₄NH(CH₂)₃NHCH₂COGluN[(CH₂)₁₇CH₃]₂
NH₂(CH₂)₃NH(CH₂)₄NH(CH₂)₃NHCH₂COGlu [N(CH₃)₂]N[(CH₂)₁₇CH₃]₂
NH₂(CH₂)₃NH(CH₂)₄NH(CH₂)₃NHCH₂COGlu [O-Bz]N[(CH₂)₁₇CH₃]₂
NH₂(CH₂)₃NH(CH₂)₄NH(CH₂)₃NHCH₂COGlu [Galactosamide]N[(CH₂)₁₇CH₃]₂
NH₂(CH₂)₃NH(CH₂)₄NH(CH₂)₃NHCH₂COGlu [Glucosamide]N[(CH₂)₁₇CH₃]₂
NH₂(CH₂)₃NH(CH₂)₄NH(CH₂)₃NHCH₂COGlu [Mannosamide]N[(CH₂)₁₇CH₃]₂
NH₂(CH₂)₃NH(CH₂)₄NH(CH₂)₃NH(CH₂)₃CON[(CH₂)₁₇CH₃]₂
NH₂(CH₂)₃NH(CH₂)₄NH(CH₂)₃NHCH₂CONH(CH₂)₅CON[(CH₂)₁₇CH₃]₂
NH₂(CH₂)₃NH(CH₂)₄NH(CH₂)₃NHCH₂COGlyN[(CH₂)₁₁CH₃]₂
NH₂(CH₂)₃NH(CH₂)₄NH(CH₂)₃NHCH₂COGlyN[(CH₂)₁₂CH₃]₂ et
NH₂(CH₂)₃NH(CH₂)₄NH(CH₂)₃NHCH₂COGlyN[(CH₂)₁₃CH₃]₂.

11. Composition pharmaceutique caractérisée en ce qu'elle contient au moins une lipopolyamine selon l'une des revendications 1 à 10 et au moins un acide nucléique.

12. Composition selon la revendication 11 caractérisée en ce que l'acide nucléique est un acide désoxyribonucléique.

13. Composition selon la revendication 11 caractérisée en ce que l'acide nucléique est un acide ribonucléique.

14. Composition selon la revendication 11, 12 ou 13 caractérisée en ce que l'acide nucléique est modifié chimiquement.

15. Composition selon l'une des revendications 11 à 14 caractérisée en ce que l'acide nucléique est un antisens.

16. Composition selon l'une des revendications 11 à 14 caractérisée en ce que l'acide nucléique comporte un gène thérapeutique.

17. Composition pharmaceutique comprenant un acide nucléique, une lipopolyamine selon l'une des revendications 1 à 6 et un adjuvant capable de s'associer au complexe lipopolyamine/acide nucléique et/ou d'améliorer son pouvoir transfectant.

18. Composition selon la revendication 17 caractérisée en ce que l'adjuvant est un ou plusieurs lipides neutres.

19. Composition selon la revendication 18 caractérisée en ce que le ou les lipides neutres sont choisis parmi les lipides synthétiques ou naturels, zwitterioniques ou dépourvus de charge ionique dans les conditions physiologiques.

20. Composition selon la revendication 19 caractérisée en ce que le ou les lipides neutres sont des lipides à 2 chaînes grasses.

21. Composition selon la revendication 19 ou 20 caractérisée en ce que le ou les lipides neutres sont choisis parmi la dioléoylphosphatidyléthanolamine (DOPE), l'oléoyl-palmitoylphos-phatidyléthanolamine (POPE), le di-stéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) et les asialogangliosides (tels que notamment les asialoGM1 et GM2).

22. Composition pharmaceutique selon l'une des revendications 11 à 21caractérisée en ce que l'adjuvant est ou comprend un composé intervenant au niveau de la condensation dudit acide nucléique

23. Composition pharmaceutique selon la revendication 22 caractérisée en ce que ledit composé dérive en tout ou partie d'une histone, d'une nucléoline et/ou d'une protamine.

24. Composition pharmaceutique selon la revendication 22 caractérisée en ce que ledit composé est constitué en tout ou partie, de motifs peptidiques (KTPKKAKKP) et/ou (ATPAKKAA) répétés de manière continue ou non, le nombre des motifs pouvant varier entre 2 et 10.

25. Composition selon l'une des revendications 11 à 24 caractérisée en ce qu'elle comprend de 0,01 à 20 équivalents d'adjuvant pour un équivalent d'acide nucléique en poids/poids et, plus préférentiellement, de 0,5 à 5.

26. Composition selon l'une quelconque des revendications 11 à 25 caractérisée en ce qu'elle comprend un véhicule pharmaceutiquement acceptable pour une formulation injectable.

27. Composition selon l'une quelconque des revendications 11 à 25 caractérisée en ce qu'elle comprend un véhicule pharmaceutiquement acceptable pour une application sur la peau et/ou les muqueuses.

28. Utilisation d'une lipopolyamine selon l'une des revendications de 1 à 10 pour la préparation d'un médicament destiné à la transfection des cellules.

29. Procédé de préparation d'une lipopolyamine selon l'une des revendications 1 à 10 caractérisé en ce qu'il met en oeuvre le couplage d'au moins une fraction lipidique à au moins une fraction polyamine assymétrique, ladite fraction polyamine ayant au préalable été obtenue par réaction bimoléculaire entre un agent alkylant lié covalemment à un support solide et une polyamine symétrique.

30. Procédé selon la revendication 29 caractérisé en ce que le couplage de la fraction lipidique à la fraction polyamine assymétrique est effectuée au niveau du support solide auquel est fixée ladite fraction polyamine assymétrique et en ce que l'on récupère ladite lipopolyamine ainsi obtenue.

31. Procédé selon la revendication 30 caractérisé en ce qu'il met en outre en oeuvre l'introduction d'agents marqueurs, de sucres ou de sondes fluorescentes sur la lipopolyamine, fixée ou non sur le support solide.

## Claims

1. Lipopolyamine, in D, L or LD form and its salts, characterized in that it is represented by the general formula I 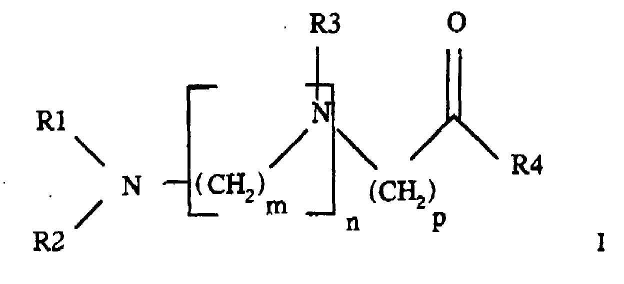 in which:
R1, R2 and R3 represent, independently of each other, a hydrogen atom or a group -(CH₂)q-NRR' with q able to range between 1, 2, 3, 4, 5 and 6, and doing so independently between the various groups R1, R2 and R3 and
R and R' representing, independently of each other, a hydrogen atom or a group -(CH₂)q'-NH₂, q' being able to range between 1, 2, 3, 4, 5 and 6, and doing so independently between the various groups R and R',
m, n and p represent, independently of each other, an integer which may vary between 0 and 6 with, when n is greater than 1, m able to take different values and R3 able to take different meanings within the general formula I, and
R4 represents a group of general formula II 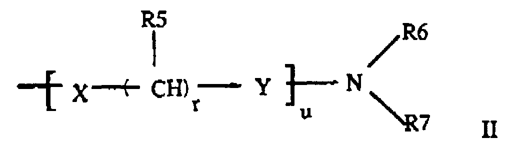 in which:
R6 and R7 represent, independently of each other, a hydrogen atom or a saturated or unsaturated C10 to C22 aliphatic radical with at least one of the two groups being other than hydrogen,
u is an integer chosen between 0 and 10 with, when u is an integer greater than 1, R5, X, Y and r able to have different meanings within the different units [X-(CHR5)r-Y]
X represents an oxygen or sulphur atom or an amine group which may or may not be monoalkylated,
Y represents a carbonyl group or a methylene group R5 represents a hydrogen atom or a side chain of a natural amino acid, which is substituted if necessary, and
r represents an integer ranging between 1 and 10 with, when r is equal to 1, R5 representing a side chain of a substituted or unsubstituted natural amino acid and, when r is greater than 1, R5 representing a hydrogen atom.

2. Lipopolyamine according to claim 1, characterized in that it is represented by one of the following subformulae 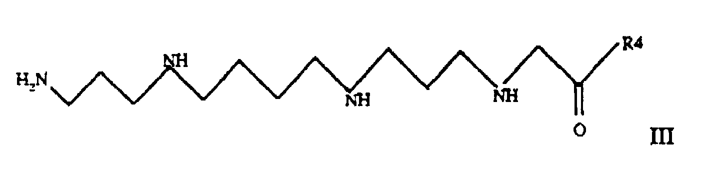 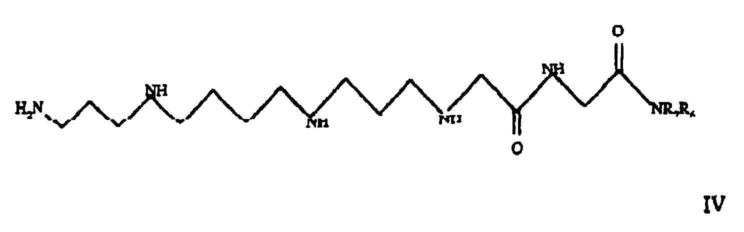 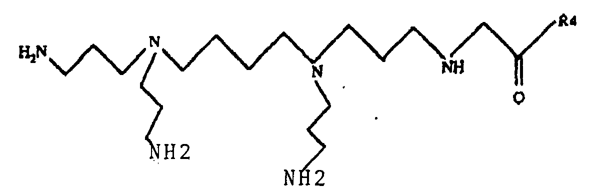 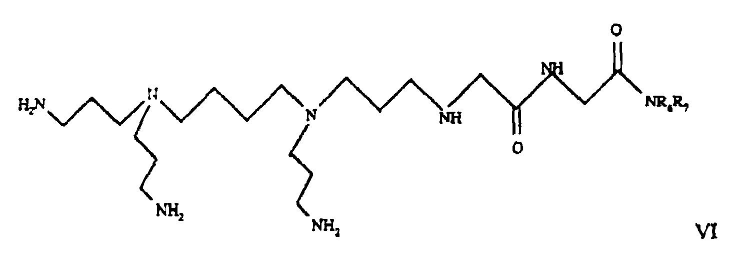 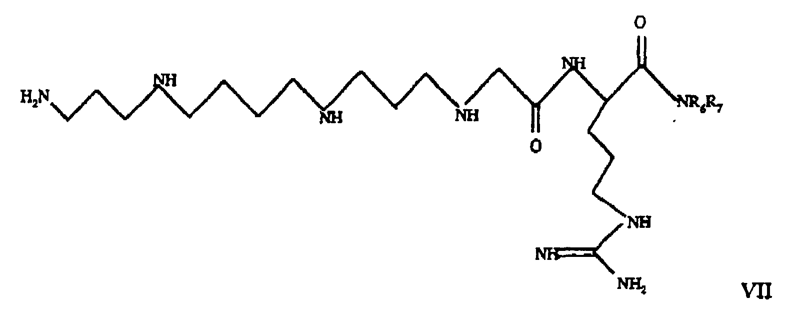 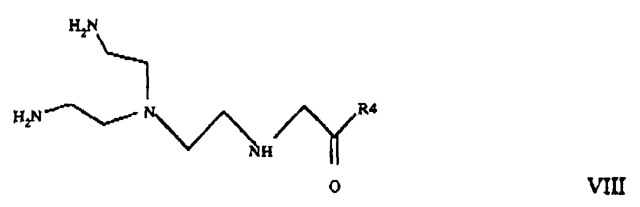 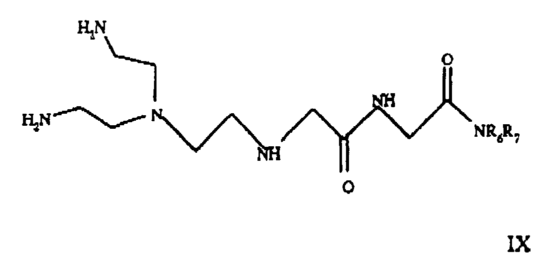 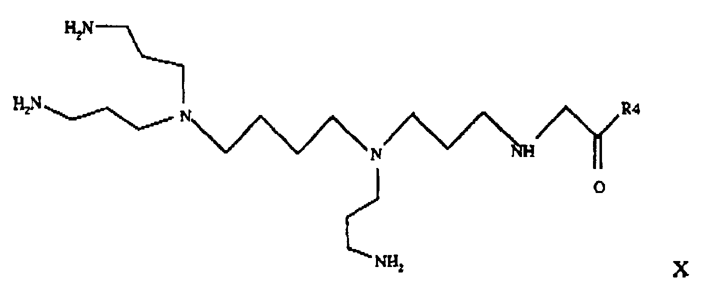 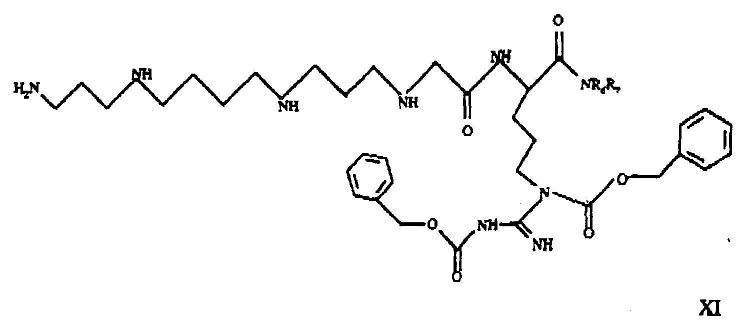 in which R4, R6 and R7 are defined as in claim 1.

3. Lipopolyamine according to claim 2, characterized in that R4 represents therein an NR6R7 group with R6 and R7 appearing in subformulae III to XII as an identical group chosen from (CH₂)₁₇CH₃, (CH₂)₁₁CH₃, (CH₂)₁₃CH₃ or (CH₂)₁₂CH₃.

4. Lipopolyamine according to one of the preceding claims, characterized in that it is combined with an extra- or intracellular targeting element.

5. Lipopolyamine according to claim 4, characterized in that it incorporates the targeting element in the amino acid side chain represented by substituent R5.

6. Lipopolyamine according to claim 4 or 5, characterized in that it is a ligand of a cell receptor present at the surface of the target cell type.

7. Lipopolyamine according to one of claims 4 to 6, characterized in that this targeting element is chosen from sugars, peptides such as antibodies or antibody fragments, cell receptor ligands or fragments thereof, receptors or receptor fragments such as ligands for growth factor receptors, for cytokine receptors, for cell lectin receptors or for adhesion protein receptors of the integrin type, for the transferrin receptor, HDL or LDL lipids and/or oligonucleotides.

8. Lipopolyamine according to either of claims 4 or 5, characterized in that this targeting element is represented by a nuclear localization signal sequence.

9. Lipopolyamine according to one of the preceding claims, characterized in that it incorporates a labelling agent of biotin, rhodamine or folate type or a linear or cyclic peptide or pseudopeptide sequence containing the Arg-Gly-Asp epitope in the amino acid side chain represented by R5.

10. Lipopolyamine according to one of the preceding claims, characterized in that it it is chosen from:
H₂N(CH₂)₃NH(CH₂)₄NH(CH₂)₃NHCH₂COGlyN[(CH₂)₁₇-CH₃]₂
H₂N(CH₂)₃NH(CH₂)₄NH(CH₂)₃NHCH₂CON[(CH₂)₁₇-CH₃]₂
H₂N(CH₂)₃NH(CH₂)₄NH(CH₂)₃NHCH₂COArgN[(CH₂)₁₇-CH₃]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(rhodamine)N[(CH₂)₁₇-CH₃]₂
H₂N(CH₂)₃NH(CH₂)₄NH(CH₂)₃NHCH₂COLys(biotinyl)N[(CH₂)₁₇-CH₃]₂
{H₂N(CH₂)₃}₂N(CH₂)₄N{(CH₂)₃NH₂}(CH₂)₃NHCH₂COGlyN[(CH₂)₁₇-CH₃]₂
{H₂N(CH₂)₃}₂N(CH₂)₄N{(CH₂)₃NH₂}(CH₂)₃NHCH₂CON[(CH₂)₁₇-CH₃]₂
{H₂N(CH₂)₂}₂N(CH₂)₂NHCH₂COGlyN[(CH₂)₁₇-CH₃]₂
{H₂N(CH₂)₂}₂N(CH₂)₂NHCH₂CON[(CH₂)₁₇-CH₃]₂
H₂N(CH₂)₃NH(CH₂)₄NH(CH₂)₃NHCH₂COGlyN[(CH₂)₁₇]₂
H₂N(CH₂)₃NH(CH₂)₄NH(CH₂)₃NHCH₂CON[(CH₂)₁₇]₂
H₂N(CH₂)₃NH(CH₂)₄NH(CH₂)₃NHCH₂COArgN[(CH₂)₁₇]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(rhodamine)N[(CH₂)₁₇-CH₃]₂
H₂N(CH₂)₃NH(CH₂)₄NH(CH₂)₃NHCH₂COLys(biotinyl)N[(CH₂)₁₇-CH₃]₂
{H₂N(CH₂)₃}₂N(CH₂)₄N{(CH₂)₃NH₂}(CH₂)₃NHCH₂COGlyN[(CH₂)₁₇-CH₃]₂
{H₂N(CH₂)₃}₂N(CH₂)₄N{(CH₂)₃NH₂}(CH₂)₃NHCH₂CON[(CH₂)₁₇-CH₃]₂
{H₂N(CH₂)₂}₂N(CH₂)₂NHCH₂COGlyN[(CH₂)₁₇-CH₃]₂
{H₂N(CH₂)₂}₂N(CH₂)₂NHCH₂CON[(CH₂)₁₇-CH₃]₂
NH₂(CH₂)₃NH(CH₂)₄N[(CH₂)₃NH₂]CH₂COGlyN[(CH₂)₁₇CH₃]₂
NH₂(CH₂)₃NH(CH₂)₄NH(CH₂)₃NHCH₂COLysN[(CH₂)₁₇CH₃]₂
NH₂(CH₂)₃NH(CH₂)₄NH(CH₂)₃NHCH₂COLys[Cl-Z]N[(CH₂)₁₇CH₃]₂
NH₂(CH₂)₃NH(CH₂)₄NH(CH₂)₃NHCH₂COLys[CHO]N[(CH₂)₁₇CH₃]₂
NH₂(CH₂)₃NH(CH₂)₄NH(CH₂)₃NHCH₂COLys[Cholesteryl]N[(CH₂)₁₇CH₃]₂
NH₂(CH₂)₃NH(CH₂)₄NH(CH₂)₃NHCH₂COLys[Arachidonyl]N[(CH₂)₁₇CH₃]₂
NH₂(CH₂)₃NH(CH₂)₄NH(CH₂)₃NHCH₂COGluN[(CH₂)₁₇CH₃]₂
NH₂(CH₂)₃NH(CH₂)₄NH(CH₂)₃NHCH₂COGlu[N(CH₃)₂]N[(CH₂)₁₇CH₃]₂
NH₂(CH₂)₃NH(CH₂)₄NH(CH₂)₃NHCH₂COGlu[O-Bz]N[(CH₂)₁₇CH₃]₂
NH₂(CH₂)₃NH(CH₂)₄NH(CH₂)₃NHCH₂COGlu[Galactosamide]N[(CH₂)₁₇CH₃]₂
NH₂(CH₂)₃NH(CH₂)₄NH(CH₂)₃NHCH₂COGlu[Glucosamide]N[(CH₂)₁₇CH₃]₂
NH₂(CH₂)₃NH(CH₂)₄NH(CH₂)₃NHCH₂COGlu[Mannosamide]N[(CH₂)₁₇CH₃]₂
NH₂(CH₂)₃NH(CH₂)₄NH(CH₂)₃NH(CH₂)₃CON[(CH₂)₁₇CH₃]₂
NH₂(CH₂)₃NH(CH₂)₄NH(CH₂)₃NHCH₂CONH(CH₂)₅CON[(CH₂)₁₇CH₃]₂
NH₂(CH₂)₃NH(CH₂)₄NH(CH₂)₃NHCH₂COGlyN[(CH₂)₁₁CH₃]₂
NH₂(CH₂)₃NH(CH₂)₄NH(CH₂)₃NHCH₂COGlyN[(CH₂)₁₂CH₃]₂ and
NH₂(CH₂)₃NH(CH₂)₄NH(CH₂)₃NHCH₂COGlyN[(CH₂)₁₃CH₃]₂.

11. Pharmaceutical composition, characterized in that it contains at least one lipopolyamine according to one of claims 1 to 10 and at least one nucleic acid.

12. Composition according to claim 11, characterized in that the nucleic acid is a deoxyribonucleic acid.

13. Composition according to claim 11, characterized in that the nucleic acid is a ribonucleic acid.

14. Composition according to claim 11, 12 or 13, characterized in that the nucleic acid is chemically modified.

15. Composition according to one of claims 11 to 14, characterized in that the nucleic acid is an antisense.

16. Composition according to one of claims 11 to 14, characterized in that the nucleic acid contains a therapeutic gene.

17. Pharmaceutical composition comprising a nucleic acid, a lipopolyamine according to one of claims 1 to 6 and an adjuvant capable of being combined with the lipopolyamine/nucleic acid complex and/or of improving its transfecting power.

18. Composition according to claim 17, characterized in that the adjuvant is one or more neutral lipids.

19. Composition according to claim 18, characterized in that the neutral lipid or lipids are chosen from natural or synthetic zwitterionic lipids or lipids lacking any ionic charge under physiological conditions.

20. Composition according to claim 19, characterized in that the neutral lipid or lipids are lipids with 2 fatty chains.

21. Composition according to claim 19 or 20, characterized in that the neutral lipid or lipids are chosen from dioleoylphosphatidylethanolamine (DOPE), oleoylpalmitoylphosphatidylethanolamine (POPE), distearoyl, -palmitoyl, -myristoyl phosphatidylethanolamine as well as derivative thereof N-methylated 1 to 3 times; phosphatidylglycerols, diacylglycerols, glycosyldiacylglycerols, cerebrosides (such as galactocerebrosides in particular), sphingolipids (such as sphingomyelins in particular) and asialogangliosides (such as asialoGM1 and GM2 in particular).

22. Pharmaceutical composition according to one of claims 11 to 21, characterized in that the adjuvant is or comprises a compound which intervenes in the condensation of the said nucleic acid.

23. Pharmaceutical composition according to claim 22, characterized in that the said compound is derived partly or totally from a histone, from a nucleoline and/or from a protamine.

24. Pharmaceutical composition according to claim 22, characterized in that the said compound consists partly or totally of peptide units (KTPKKAKKP) and/or (ATPAKKAA) repeated continuously or non-continuously, it being possible for the number of units to range between 2 and 10.

25. Composition according to one of claims 11 to 24, characterized in that it comprises from 0.01 to 20 equivalents of adjuvant per one equivalent of nucleic acid on a weight/weight basis and, more preferably, from 0.5 to 5.

26. Composition according to any one of claims 11 to 25, characterized in that it comprises a vehicle which is pharmaceutically acceptable for an injectable formulation.

27. Composition according to any one of claims 11 to 25, characterized in that it comprises a vehicle which is pharmaceutically acceptable for an application to the skin and/or the mucous membranes.

28. Use of a lipopolyamine according to one of claims 1 to 10 for the preparation of a medicinal product intended for the transfection of cells.

29. Preparation process of a lipopolyamine according to one of claims 1 to 10, characterized in that it uses the coupling of at least one lipid fraction with at least one asymmetric polyamine fraction, the said polyamine fraction having previously been obtained by bimolecular reaction between an alkylating agent covalently attached to a solid support and a symmetric polyamine.

30. Process according to claim 29, characterized in that the coupling of the lipid fraction with the asymmetric polyamine fraction is carried out on the solid support to which the said asymmetric polyamine fraction is bound and in that the said lipopolyamine thus obtained is recovered.

31. Process according to claim 30, characterized in that it also involves the introduction of labelling agents, sugars or fluorescent probes onto the lipopolyamine, which is or is not bound to the solid support.

## Patentansprüche

1. Lipopolyamin in der Form D, L oder LD und seine Salze, dadurch gekennzeichnet, daß es durch die folgende allgemeine Formel (I) dargestellt wird: 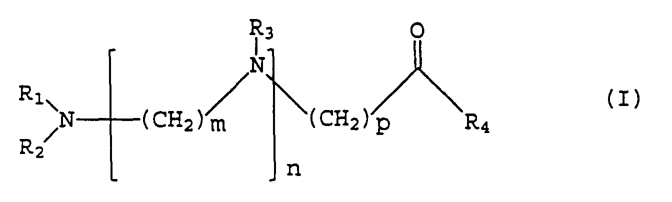 in der
R₁, R₂ und R₃ unabhängig voneinander ein Wasserstoffatom oder eine Gruppe-(CH₂)_{q}-NRR' darstellen, mit
q, das zwischen 1, 2, 3, 4, 5 oder 6 in unabhängiger Weise zwischen den verschiedenen Gruppen R₁, R₂ und R₃ variieren kann, und
R und R', die unabhängig voneinander ein Wasserstoffatom oder eine Gruppe -(CH₂)_{q'}-NH₂ bedeuten, wobei q' zwischen 1, 2, 3, 4, 5 oder 6 in unabhängiger Weise zwischen den verschiedenen Gruppen R und R' variieren kann,
m, n und p, die unabhängig voneinander eine ganze Zahl darstellen, die zwischen 0 und 6 variieren kann, und wenn n höher als 1 ist, m verschiedene Werte und R₃ verschiedene Bedeutungen in der allgemeinen Formel (I) annehmen können, und
R₄, das eine Gruppe der allgemeinen Formel (II) 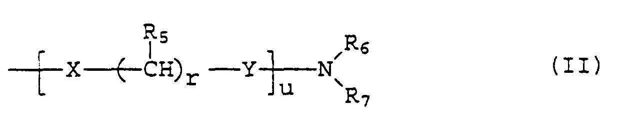 darstellt, in der
R₆ und R₇ unabhängig voneinander ein Wasserstoffatom oder einen gesättigten oder ungesättigten aliphatischen Rest mit 10 bis 22 Kohlenstoffatomen bedeuten, wobei mindestens eine der beiden Gruppen von Wasserstoff verschieden ist,
u eine ganze Zahl zwischen 0 und 10 darstellt, und wenn u eine ganze Zahl höher als 1 ist, R₅, X, Y und r verschiedene Bedeutungen in den verschiedenen Struktureinheiten [X-(CHR₅)ᵣ-Y] besitzen können,
X ein Atom von Sauerstoff oder Schwefel oder eine Gruppe Amino, monoalkyliert oder nicht, bedeutet,
Y eine Gruppe Carbonyl oder eine Gruppe Methylen darstellt,
R₅ ein Wasserstoffatom oder eine Seitenkette einer natürlichen Aminosäure, gegebenenfalls substituiert, bedeutet, und
r eine ganze Zahl ist, die zwischen 1 und 10 variiert, und wenn r gleich 1 ist, R₅ eine Seitenkette einer natürlichen Aminosäure, substituiert oder nicht, darstellt und wenn r höher als 1 ist, R₅ ein Wasserstoffatom bedeutet.

2. Lipopolyamin nach Anspruch 1, dadurch gekennzeichnet, daß es durch eine der folgenden Formeln dargestellt wird: 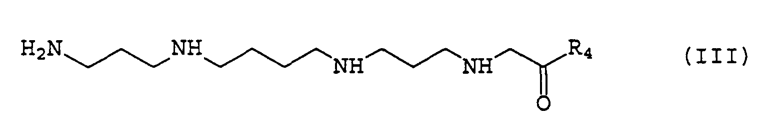 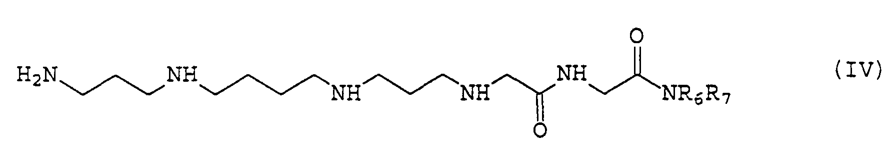 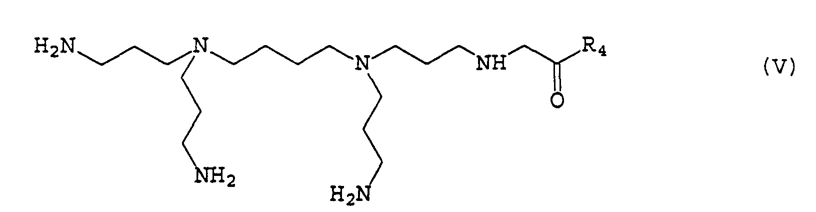 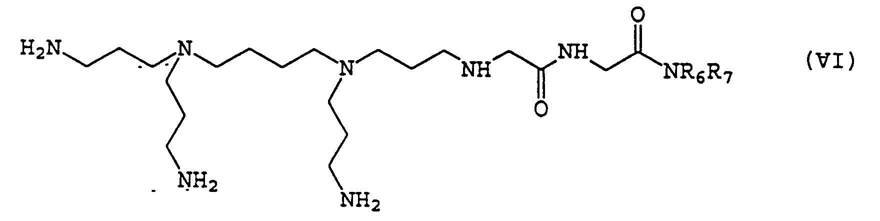 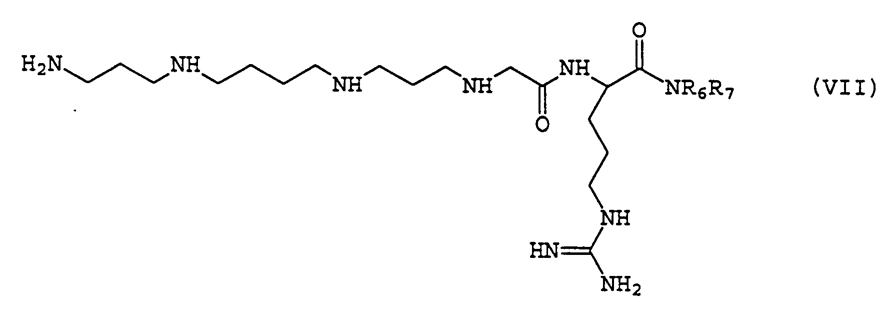 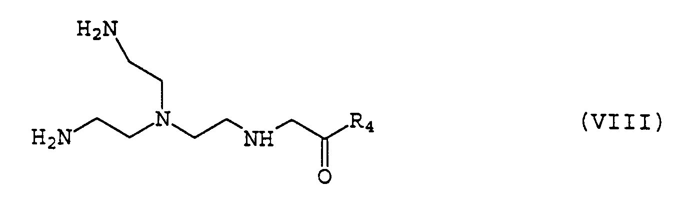 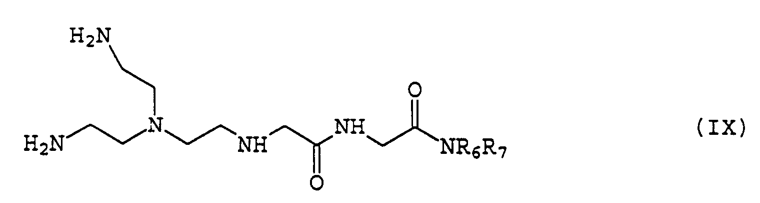 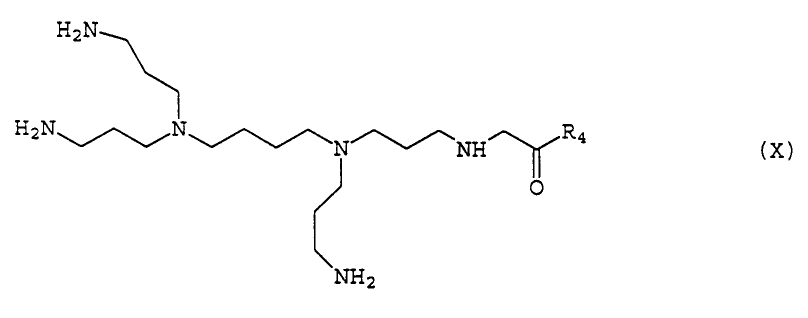 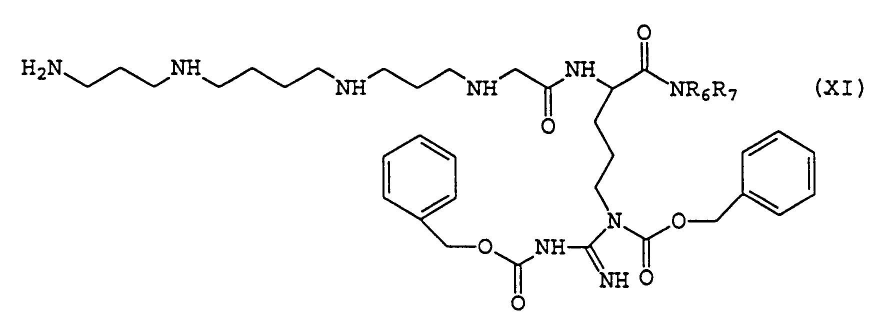 in denen R₄, R₆ und R₇ wie in Anspruch 1 definiert sind.

3. Lipopolyamin nach Anspruch 2, dadurch gekennzeichnet, daß darin R₄ eine Gruppe NR₆R₇ darstellt, mit R₆ und R₇, wie sie in den Unterformeln (III) bis (XII) eine identische Gruppe bedeuten, ausgewählt unter (CH₂)₁₇CH₃, (CH₂)₁₁CH₃, (CH₂)₁₃CH₃ oder (CH₂)₁₂CH₃.

4. Lipopolyamin nach einem der vorstehenden Ansprüche, dadurch gekennzeichnet, daß es mit einem extrazellulären oder intrazellulären Zielelement assoziiert ist.

5. Lipopolyamin nach Anspruch 4, dadurch gekennzeichnet, daß es das Zielelement im Bereich der durch den Substituenten R₅ dargestellten Aminosäure-Seitenkette inkorporiert.

6. Lipopolyamin nach Anspruch 4 oder 5, dadurch gekennzeichnet, daß es sich um einen zellulären Rezeptor-Liganden handelt, der sich an der Oberfläche des zellulären Zieltyps befindet.

7. Lipopolyamin nach einem der Ansprüche 4 bis 6, dadurch gekennzeichnet, daß dieses Zielelement unter Zuckern, Peptiden wie Antikörpern oder Fragmenten von Antikörpern, zellulären Rezeptor-Liganden oder deren Fragmenten, Rezeptoren oder Fragmenten von Rezeptoren wie Liganden von Rezeptoren der Wachstumsfaktoren, von Rezeptoren der Cytokine, von Rezeptoren der zellulären Lectine oder von Rezeptoren der Adhäsionsproteine vom Typ der Integrine, vom Rezeptor des Transferrins, von Lipiden HDL oder LDL und/oder Oligonucleotiden ausgewählt wird.

8. Lipopolyamin nach einem der Ansprüche 4 oder 5, dadurch gekennzeichnet, daß dieses Zielelement durch eine Signalsequenz der nuklearen Lokalisation dargestellt wird.

9. Lipopolyamin nach einem der vorstehenden Ansprüche, dadurch gekennzeichnet, daß es ein Markierungsmittel vom Typ Biotin, Rhodamin, Folat oder eine lineare oder cyclische peptidische oder pseudopeptidische Sequenz inkorporiert, die das Epitop Arg-Gly-Asp im Bereich der durch R₅ dargestellten Aminosäure-Seitenkette umfaßt.

10. Lipopolyamin nach einem der vorstehenden Ansprüche, dadurch gekennzeichnet, daß es ausgewählt wird unter:
H₂N(CH₂)₃NH(CH₂)₄NH(CH₂)₃NHCH₂COGlyN[(CH₂)₁₇-CH₃]₂
H₂N(CH₂)₃NH(CH₂)₄NH(CH₂)₃NHCH₂CON[(CH₂)₁₇-CH₃]₂
H₂N(CH₂)₃NH(CH₂)₄NH(CH₂)₃NHCH₂COArgN[(CH₂)₁₇-CH₃]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(Rhodamin)N[(CH₂)₁₇-CH₃]₂
H₂N(CH₂)₃NH(CH₂)₄NH(CH₂)₃NHCH₂COLys(Biotinyl)N[(CH₂)₁₇-CH₃]₂
[H₂N(CH₂)₃]₂N(CH₂)₄N[(CH₂)₃NH₂](CH₂)₃NHCH₂COGlyN[(CH₂)₁₇-CH₃]₂
[H₂N(CH₂)₃]₂N(CH₂)₄N[(CH₂)₃NH₂](CH₂)₃NHCH₂CON[(CH₂)₁₇-CH₃]₂
[H₂N(CH₂)₂]₂N(CH₂)₂NHCH₂COGlyN[(CH₂)₁₇-CH₃]₂
[H₂N(CH₂)₂]₂N(CH₂)₂NHCH₂CON[(CH₂)₁₇-CH₃]₂
H₂N(CH₂)₃NH(CH₂)₄NH(CH₂)₃NHCH₂COGlyN[(CH₂)₁₇]₂
H₂N(CH₂)₃NH(CH₂)₄NH(CH₂)₃NHCH₂CON[(CH₂)₁₇]₂
H₂N(CH₂)₃NH(CH₂)₄NH(CH₂)₃NHCH₂COArgN[(CH₂)₁₇]₂
H₂N(CH₂)₃NH(CH₂)₄NH(CH₂)₃NHCH₂COArg(Z)₂N[(CH₂)₁₇-CH₃]₂
H₂N(CH₂)₃NH(CH₂)₄NH(CH₂)₃NHCH₂COLys(Rhodamin)N[(CH₂)₁₇-CH₃]₂
H₂N(CH₂)₃NH(CH₂)₄NH(CH₂)₃NHCH₂COLys(Biotinyl)N[(CH₂)₁₇-CH₃]₂
[H₂N(CH₂)₃]₂N(CH₂)₄N[(CH₂)₃NH₂](CH₂)₃NHCH₂COGlyN[(CH₂)₁₇-CH₃]₂
[H₂N(CH₂)₃]₂N(CH₂)₄N[(CH₂)₃NH₂](CH₂)₃NHCH₂CON[(CH₂)₁₇-CH₃]₂
[H₂N(CH₂)₂]₂N(CH₂)₂NHCH₂COGlyN[(CH₂)₁₇-CH₃]₂
[H₂N(CH₂)₂]₂N(CH₂)₂NHCH₂CON[(CH₂)₁₇-CH₃]₂
NH₂(CH₂)₃NH(CH₂)₄N[(CH₂)₃NH₂]CH₂COGlyN[(CH₂)₁₇CH₃]₂
NH₂(CH₂)₃NH(CH₂)₄NH(CH₂)₃NHCH₂COLysN[(CH₂)₁₇CH₃]₂
NH₂(CH₂)₃NH(CH₂)₄NH(CH₂)₃NHCH₂COLys[Cl-Z]N[(CH₂)₁₇CH₃]₂
NH₂(CH₂)₃NH(CH₂)₄NH(CH₂)₃NHCH₂COLys[CHO]N[(CH₂)₁₇CH₃]₂
NH₂(CH₂)₃NH(CH₂)₄NH(CH₂)₃NHCH₂COLys[Cholesteryl]N[(CH₂)₁₇CH₃]₂
NH₂(CH₂)₃NH(CH₂)₄NH(CH₂)₃NHCH₂COLys[Arachinodyl]N[(CH₂)₁₇CH₃]₂
NH₂(CH₂)₃NH(CH₂)₄NH(CH₂)₃NHCH₂COGluN[(CH₂)₁₇CH₃]₂
NH₂(CH₂)₃NH(CH₂)₄NH(CH₂)₃NHCH₂COGlu[N(CH₃)₂]N[(CH₂)₁₇CH₃]₂
NH₂(CH₂)₃NH(CH₂)₄NH(CH₂)₃NHCH₂COGlu[O-Bz]N[(CH₂)₁₇CH₃]₂
NH₂(CH₂)₃NH(CH₂)₄NH(CH₂)₃NHCH₂COGlu[Galactosamid]N[(CH₂)₁₇CH₃]₂
NH₂(CH₂)₃NH(CH₂)₄NH(CH₂)₃NHCH₂COGlu[Glucosamid]N[(CH₂)₁₇CH₃]₂
NH₂(CH₂)₃NH(CH₂)₄NH(CH₂)₃NHCH₂COGlu[Mannosamid]N[(CH₂)₁₇CH₃]₂
NH₂(CH₂)₃NH(CH₂)₄NH(CH₂)₃NH(CH₂)₃CON[(CH₂)₁₇CH₃]₂
NH₂(CH₂)₃NH(CH₂)₄NH(CH₂)₃NHCH₂CONH(CH₂)₅CON[(CH₂)₁₇CH₃]₂
NH₂(CH₂)₃NH(CH₂)₄NH(CH₂)₃NHCH₂COGlyN[(CH₂)₁₁CH₃]₂
NH₂(CH₂)₃NH(CH₂)₄NH(CH₂)₃NHCH₂COGlyN[(CH₂)₁₂CH₃]₂ und
NH₂(CH₂)₃NH(CH₂)₄NH(CH₂)₃NHCH₂COGlyN[(CH₂)₁₃CH₃]₂.

11. Pharmazeutische Zusammensetzung, dadurch gekennzeichnet, daß sie mindestens ein Lipopolyamin nach einem der Ansprüche 1 bis 10 und mindestens eine Nucleinsäure enthält.

12. Zusammensetzung nach Anspruch 11, dadurch gekennzeichnet, daß die Nucleinsäure eine Desoxyribonucleinsäure ist.

13. Zusammensetzung nach Anspruch 11, dadurch gekennzeichnet, daß die Nucleinsäure eine Ribonucleinsäure ist.

14. Zusammensetzung nach Anspruch 11, 12 oder 13, dadurch gekennzeichnet, daß die Nucleinsäure chemisch modifiziert ist.

15. Zusammensetzung nach einem der Ansprüche 11 bis 14, dadurch gekennzeichnet, daß die Nucleinsäure eine Antisense-Nuclein säure ist.

16. Zusammensetzung nach einem der Ansprüche 11 bis 14, dadurch gekennzeichnet, daß die Nucleinsäure ein therapeutisches Gen umfaßt.

17. Pharmazeutische Zusammensetzung, umfassend eine Nucleinsäure, ein Lipopolyamin nach einem der Ansprüche 1 bis 6 und einen Zusatzstoff, der fähig ist, sich an den Komplex Lipopolyamin/Nucleinsäure zu assoziieren und/oder dessen Transfektionsvermögen zu verbessern.

18. Zusammensetzung nach Anspruch 17, dadurch gekennzeichnet, daß der Zusatzstoff aus einem oder mehreren neutralen Lipiden besteht.

19. Zusammensetzung nach Anspruch 18, dadurch gekennzeichnet, daß das oder die neutralen Lipide unter den synthetischen oder natürlichen, zwitterionischen oder den Lipiden ausgewählt werden, die unter physiologischen Bedingungen von ionischer Ladung frei sind.

20. Zusammensetzung nach Anspruch 19, dadurch gekennzeichnet, daß das oder die neutralen Lipide solche mit zwei Fettketten sind.

21. Zusammensetzung nach Anspruch 19 oder 20, dadurch gekennzeichnet, daß das oder die neutralen Lipide unter Dioleylphosphatidyl-ethanolamin (DOPE), Oleyl-palmitoylphosphatidyl-ethanolamin (POPE), Di-stearoyl, -palmitoyl, -myristoyl phosphatidyl-ethanolamin sowie ihren ein- bis dreimal N-methylierten Derivaten; den Phosphatidyl-glycerinen, den Diacyl-glycerinen, den Glycosyldiacyl-glycerinen, den Cerebrosiden (wie insbesondere den Galactocerebrosiden), den Sphingolipiden (wie insbesondere den Sphingomyelinen) und den Asialogangliosiden (wie insbesondere den asialoGMl und GM2) ausgewählt werden.

22. Pharmazeutische Zusammensetzung nach einem der Ansprüche 11 bis 21, dadurch gekennzeichnet, daß der Zusatzstoff eine Verbindung ist oder eine Verbindung umfaßt, die im Bereich der Kondensation der genannten Nucleinsäure eingreift.

23. Pharmazeutische Zusammensetzung nach Anspruch 22, dadurch gekennzeichnet, daß sich die genannte Verbindung vollständig oder teilweise von einem Histon, einem Nucleolin und/oder einem Protamin ableitet.

24. Pharmazeutische Zusammensetzung nach Anspruch 22, dadurch gekennzeichnet, daß die genannte Verbindung vollständig oder teilweise aus peptidischen Struktureinheiten (KTPKKAKKP) und/oder (ATPAKKAA) besteht, wiederkehrend in kontinuierlicher Weise oder nicht, wobei die Anzahl der Struktureinheiten zwischen 2 und 10 variieren kann.

25. Zusammensetzung nach einem der Ansprüche 11 bis 24, dadurch gekennzeichnet, daß sie 0,01 bis 20 Äquivalente Zusatzstoff pro Äquivalent Nucleinsäure (in Gew./Gew.) und vorzugsweise 0,5 bis 5 Äquivalente umfaßt.

26. Zusammensetzung nach irgendeinem der Ansprüche 11 bis 25, dadurch gekennzeichnet, daß sie einen für eine injizierbare Formulierung pharmazeutisch akzeptablen Füllstoff umfaßt.

27. Zusammensetzung nach irgendeinem der Ansprüche 11 bis 25, dadurch gekennzeichnet, daß sie einen für eine Anwendung auf der Haut und/oder den Schleimhäuten pharmazeutisch akzeptablen Füllstoff umfaßt.

28. Verwendung eines Lipopolyamins nach einem der Ansprüche 1 bis 10 zur Herstellung eines Arzneimittels, das für die Transfektion von Zellen vorgesehen ist.

29. Verfahren zur Herstellung eines Lipopolyamins nach einem der Ansprüche 1 bis 10, dadurch gekennzeichnet, daß die Kupplung von mindestens einer lipidischen Fraktion an mindestens eine asymmetrische Polyaminfraktion durchgeführt wird, wobei die genannte Polyaminfraktion zuvor durch biomolekulare Reaktion zwischen einem Alkylierungsmittel, das kovalent an einen festen Träger gebunden ist, und einem symmetrischen Polyamin erhalten wurde.

30. Verfahren nach Anspruch 29, dadurch gekennzeichnet, daß die Kupplung der lipidischen Fraktion an die asymmetrische Polyaminfraktion im Bereich des festen Trägers durchgeführt wird, an den die genannte asymmetrische Polyaminfraktion fixiert ist, und dadurch, daß man das genannte, auf diese Weise erhaltene Lipopolyamin gewinnt.

31. Verfahren nach Anspruch 30, dadurch gekennzeichnet, daß außerdem das Einbringen von Markierungsmitteln, Zuckern oder Fluoreszenzsonden in das Lipopolyamin durchgeführt wird, das an den festen Träger fixiert ist oder nicht.
